(19) Europäisches Patentamt
European Patent Office
Office européen des brevets

(11) EP 1 633 724 B1

(12) EUROPEAN PATENT SPECIFICATION

(45) Date of publication and mention of the grant of the patent:
**04.05.2011 Bulletin 2011/18**

(21) Application number: **04720068.8**

(22) Date of filing: **12.03.2004**

(51) Int Cl.:
*C07D 237/32* (2006.01) *C07D 417/12* (2006.01) *C07D 401/12* (2006.01) *C07D 409/12* (2006.01) *C07D 403/12* (2006.01) *C07D 413/12* (2006.01) *C07D 407/12* (2006.01) *A61K 31/502* (2006.01) *A61P 31/20* (2006.01)

(86) International application number:
**PCT/GB2004/001059**

(87) International publication number:
**WO 2004/080976 (23.09.2004 Gazette 2004/39)**

# (54) PHTHALAZINONE DERIVATIVES

PHTHALAZINONDERIVATIVE

DERIVES DE PHTALAZINONE

(84) Designated Contracting States:
**AT BE BG CH CY CZ DE DK EE ES FI FR GR HU IE IT LI LU MC NL PL PT RO SE SI SK TR**
Designated Extension States:
**LT LV**

(30) Priority: **12.03.2003 GB 0305681**
**14.03.2003 US 454995 P**
**06.08.2003 US 493399 P**
**01.12.2003 US 526244 P**

(43) Date of publication of application:
**15.03.2006 Bulletin 2006/11**

(73) Proprietors:
- **Kudos Pharmaceuticals Limited London W2 6BD (GB)**
- **Maybridge Limited Tintagel, Cornwall PL34 0HW (GB)**

(72) Inventors:
- **MARTIN, Niall, Morrison, Barr Cambridge, Cambridgeshire CB4 0WG (GB)**
- **SMITH, Graeme, Cameron, Murray Cambridge, Cambridgeshire CB4 0WG (GB)**
- **JACKSON, Stephen, Philip Cambridge, Cambridgeshire CB4 0WG (GB)**
- **LOH, Vincent, Junior, M. Horsham, Sussex RH13 5PX (GB)**
- **COCKCROFT, Xiao-Ling, Fan Horsham, Sussex RH13 5PX (GB)**
- **MATTHEWS, Ian, Timothy, Williams Horsham, Sussex RH13 5PX (GB)**
- **MENEAR, Keith, Allan Horsham, Sussex RH13 5PX (GB)**
- **KERRIGAN, Frank Tintagel, Cornwall PL34 0HW (GB)**
- **ASHWORTH, Alan Greater London, London SW3 6JB (GB)**

(74) Representative: **Watson, Robert James et al Mewburn Ellis LLP 33 Gutter Lane London EC2V 8AS (GB)**

(56) References cited:
**WO-A-02/36576 WO-A-02/090334**
**WO-A-03/093261**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to phthalazinone derivatives, and their use as pharmaceuticals. In particular, the present invention relates to the use of these compounds to inhibit the activity of the enzyme poly (ADP-ribose)polymerase, also known as poly(ADP-ribose)synthase and poly ADP-ribosyltransferase, and commonly referred to as PARP.

The mammalian enzyme PARP (a 113-kDa multidomain protein) has been implicated in the signalling of DNA damage through its ability to recognize and rapidly bind to DNA single or double strand breaks (D'Amours, et al., Biochem. J., 342, 249-268 (1999)).

Several observations have led to the conclusion that PARP participates in a variety of DNA-related functions including gene amplification, cell division, differentiation, apoptosis, DNA base excision repair and also effects on telomere length and chromosome stability (d'Adda di Fagagna, et al., Nature Gen., 23(1), 76-80 (1999)).

Studies on the mechanism by which PARP modulates DNA repair and other processes has identified its importance in the formation of poly (ADP-ribose) chains within the cellular nucleus (Althaus, F.R. and Richter, C., ADP-Ribosylation of Proteins: Enzymology and Biological Significance, Springer-Verlag, Berlin (1987)). The DNA-bound, activated PARP utilizes NAD to synthesize poly (ADP-ribose) on a variety of nuclear target proteins, including topoisomerase, histones and PARP itself (Rhun, et al., Biochem. Biophys. Res. Commun., 245, 1-10 (1998))

Poly (ADP-ribosyl)ation has also been associated with malignant transformation. For example, PARP activity is higher in the isolated nuclei of SV40-transformed fibroblasts, while both leukemic cells and colon cancer cells show higher enzyme activity than the equivalent normal leukocytes and colon mucosa (Miwa, et al., Arch. Biochem. Biophys., 181, 313-321 (1977); Burzio, et al., Proc. Soc. Exp. Bioi. Med., 149, 933-938 (1975); and Hirai, et al., Cancer Res., 43, 3441-3446 (1983)).

A number of low-molecular-weight inhibitors of PARP have been used to elucidate the functional role of poly (ADP-ribosyl)ation in DNA repair. In cells treated with alkylating agents, the inhibition of PARP leads to a marked increase in DNA-strand breakage and cell killing (Durkacz, et al., Nature, 283, 593-596 (1980); Berger, N.A., Radiation Research, 101, 4-14 (1985)).

Subsequently, such inhibitors have been shown to enhance the effects of radiation response by suppressing the repair of potentially lethal damage (Ben-Hur, et al., British Journal of Cancer, 49* (Suppl. VI), 34-42 (1984); Schlicker, et al., Int. J. Radiat. Bioi., 75, 91-100 (1999)). PARP inhibitors have been reported to be effective in radio sensitising hypoxic tumour cells (US 5,032,617; US 5,215,738 and US 5, 091, 653) .

Furthermore, PARP knockout (PARP -/-) animals exhibit genomic instability in response to alkylating agents and γ-irradiation (Wang, et al., Genes Dev., 9, 509-520 (1995); Menissier de Murcia, et al., Proc. Natl. Acad. Sci. USA, 94, 7303-7307 (1997)).

A role for PARP has also been demonstrated in certain vascular diseases, septic shock, ischaemic injury and neurotoxicity (Cantoni, et al., Biochim. Biophys. Acta, 1014, 1-7 (1989); Szabo, et al., J. Clin. Invest., 100, 723-735 (1997)). Oxygen radical DNA damage that leads to strand breaks in DNA, which are subsequently recognised by PARP, is a major contributing factor to such disease states as shown by PARP inhibitor studies (Cosi, et al., J. Neurosci. Res., 39, 38-46 (1994); Said, et al., Proc. Natl. Acad. Sci. U.S.A., 93, 4688-4692 (1996)). More recently, PARP has been demonstrated to play a role in the pathogenesis of haemorrhagic shock (Liaudet, et al., Proc. Natl. Acad. Sci. U.S.A., 97(3), 10203-10208 (2000)).

It has also been demonstrated that efficient retroviral infection of mammalian cells is blocked by the inhibition of PARP activity. Such inhibition of recombinant retroviral vector infections was shown to occur in various different cell types (Gaken, et al., J. Virology, 70(6), 3992-4000 (1996)). Inhibitors of PARP have thus been developed for the use in anti-viral therapies and in cancer treatment (WO 91/18591).

Moreover, PARP inhibition has been speculated to delay the onset of aging characteristics in human fibroblasts (Rattan and Clark, Biochem. Biophys. Res. Comm., 201(2), 665-672 (1994)). This may be related to the role that PARP plays in controlling telomere function (d'Adda di Fagagna, et al., Nature Gen., 23(1), 76-80 (1999)).

Some of the present inventors have previously described (WO 02/36576) a class of 1(2H)-phthalazinone compounds which act as PARP inhibitors. The compounds have the general formula:

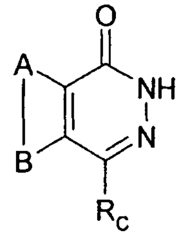

where A and B together represent an optionally substituted, fused aromatic ring and where $R_c$ is represented by -L-$R_L$. A large number of examples are of the formula:

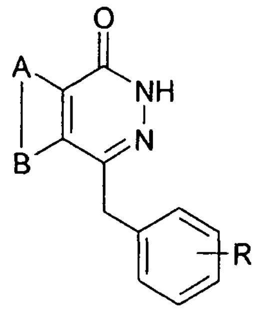

where R represent one or more optional substituents.

The present inventors have now discovered that compounds where R is of a certain nature exhibit surprising levels of inhibition of the activity of PARP, and/or of potentiation of tumour cells to radiotherapy and various chemotherapies.

Accordingly, the first aspect of the present invention provides a compound of the formula (**I**):

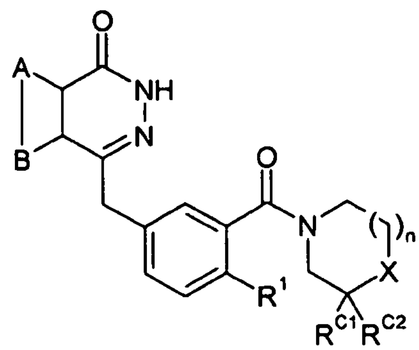

and isomers, salts and solvates thereof, wherein:

A and B together represent a fused benzene ring, optionally substituted by one or more groups selected from halo, nitro, hydroxy, ether, thiol, thioether, amino, $C_{1-7}$ alkyl, $C_{3-20}$ heterocyclyl and $C_{5-20}$ aryl;
X can be $NR^X$ or $CR^XR^Y$;
if X = $NR^X$ then n is 1 or 2 and if X = $CR^XR^Y$ then n is 1;
$R^X$ is selected from the group consisting of H, optionally substituted $C_{1-20}$ alkyl, $C_{5-20}$ aryl, $C_{3-20}$ heterocyclyl, amido, thioamido, ester, -C(=O)$R^z$, and sulfonyl groups, wherein the optional substituents are selected from $C_{1-20}$ alkyl, $C_{5-20}$ aryl, $C_{3-20}$ heterocyclyl, halo, hydroxy, ether, nitro, cyano, -C(=O)$R^Z$, carboxy, ester, amido, acylamido, ureido, acyloxy, thiol, thioether, sulfoxide, sulfonyl, thioamido and sulfonamino;
$R^Y$ is selected from H, hydroxy, amino;
or $R^X$ and $R^Y$ may together form a spiro-$C_{3-7}$ cycloalkyl or heterocyclyl group;
$R^{C1}$ and $R^{C2}$ are both hydrogen; and
$R^1$ is selected from H and halo,
wherein $R^Z$ is selected from H, $C_{1-7}$ alkyl, $C_{3-20}$ heterocyclyl or a $C_{5-20}$ aryl.

Therefore, if X is $CR^XR^Y$, then n is 1, the compound is of formula (**Ia**):

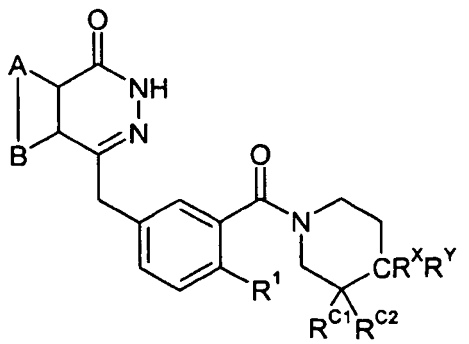

(Ia)

If X is $NR^X$, and n is 1, the compound is of formula (**Ib**):

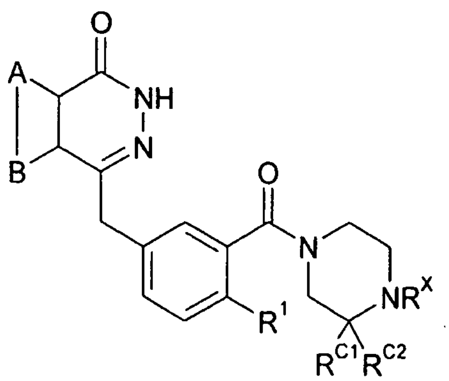

(Ib)

If X is $NR^X$, and n is 2, the compound is of formula (**Ic**):

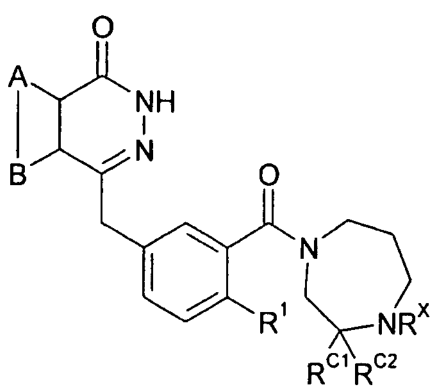

(Ic)

A second aspect of the present invention provides a pharmaceutical composition comprising a compound of the first aspect and a pharmaceutically acceptable carrier or diluent.

A third aspect of the present invention provides a compound of the first aspect for use in a method of treatment of the human or animal body.

A fourth aspect of the present invention provides the use of a compound as defined in the first aspect of the invention in the preparation of a medicament for inhibiting the activity of PARP.

Further aspects of the invention provide the use of a compound as defined in the first aspect of the invention in the preparation of a medicament for the treatment of: vascular disease; septic shock; ischaemic injury; neurotoxicity; haemorrhagic shock; viral infection; or diseases ameliorated by the inhibition of the activity of PARP. Another further aspect of the invention provides for the use of a compound as defined in the first aspect of the invention in the preparation of a medicament for use as an adjunct in cancer therapy or for potentiating tumour cells for treatment with ionizing

radiation or chemotherapeutic agents.

In further aspects of the present invention, the compounds may be used in the preparation of a medicament for the treatment of cancer which is deficient in Homologous Recombination (HR) dependent DNA DSB repair activity, or in the treatment of a patient of a cancer which is deficient in HR dependent DNA DSB repair activity, comprising administering to said patient a therapeutically-effective amount of the compound.

The HR dependent DNA DSB repair pathway repairs double-strand breaks (DSBs) in DNA via homologous mechanisms to reform a continuous DNA helix (K.K. Khanna and S.P. Jackson, Nat. Genet. 27(3): 247-254 (2001)). The components of the HR dependent DNA DSB repair pathway include, but are not limited to, ATM (NM 000051), RAD51 (NM_002875), RAD51L1 (NM_002877), RAD51C (NM_002876), RAD51L3 (NM_002878), DMC1 (NM_007068), XRCC2 (NM_005431), XRCC3 (NM_005432), RAD52 (NM_002879), RAD54L (NM_003579), RAD54B (NM_012415), BRCA1 (NM_007295), BRCA2 (NM_000059), RAD50 (NM_005732), MRE11A (NM_005590) and NBS1 (NM_002985). Other proteins involved in the HR dependent DNA DSB repair pathway include regulatory factors such as EMSY (Hughes-Davies, et al., Cell, 115, pp523-535). HR components are also described in Wood, et al., Science, 291, 1284-1289 (2001).

A cancer which is deficient in HR dependent DNA DSB repair may comprise or consist of one or more cancer cells which have a reduced or abrogated ability to repair DNA DSBs through that pathway, relative to normal cells i.e. the activity of the HR dependent DNA DSB repair pathway may be reduced or abolished in the one or more cancer cells.

The activity of one or more components of the HR dependent DNA DSB repair pathway may be abolished in the one or more cancer cells of an individual having a cancer which is deficient in HR dependent DNA DSB repair. Components of the HR dependent DNA DSB repair pathway are well characterised in the art (see for example, Wood, et al., Science, 291, 1284-1289 (2001)) and include the components listed above.

In some preferred embodiments, the cancer cells may have a BRCA1 and/or a BRCA2 deficient phenotype i.e. BRCA1 and/or BRCA2 activity is reduced or abolished in the cancer cells. Cancer cells with this phenotype may be deficient in BRCA1 and/or BRCA2, i.e. expression and/or activity of BRCA1 and/or BRCA2 may be reduced or abolished in the cancer cells, for example by means of mutation or polymorphism in the encoding nucleic acid, or by means of amplification, mutation or polymorphism in a gene encoding a regulatory factor, for example the EMSY gene which encodes a BRCA2 regulatory factor (Hughes-Davies, et al., Cell, 115, 523-535).

BRCA1 and BRCA2 are known tumour suppressors whose wild-type alleles are frequently lost in tumours of heterozygous carriers (Jasin M., Oncogene, 21(58), 8981-93 (2002); Tutt, et al., Trends Mol Med., 8(12), 571-6, (2002)). The association of BRCA1 and/or BRCA2 mutations with breast cancer is well-characterised in the art (Radice, P.J., Exp Clin Cancer Res., 21(3 Suppl), 9-12 (2002)). Amplification of the EMSY gene, which encodes a BRCA2 binding factor, is also known to be associated with breast and ovarian cancer.

Carriers of mutations in BRCA1 and/or BRCA2 are also at elevated risk of cancer of the ovary, prostate and pancreas.

In some preferred embodiments, the individual is heterozygous for one or more variations, such as mutations and polymorphisms, in BRCA1 and/or BRCA2 or a regulator thereof. The detection of variation in BRCA1 and BRCA2 is well-known in the art and is described, for example in EP 699 754, EP 705 903, Neuhausen, S.L. and Ostrander, E.A., Genet. Test, 1, 75-83 (1992); Chappnis, P.O. and Foulkes, W.D., Cancer Treat Res, 107, 29-59 (2002); Janatova M., et al., Neoplasma, 50(4), 246-50 (2003); Jancarkova, N., Ceska Gynekol., 68(1), 11-6 (2003)). Determination of amplification of the BRCA2 binding factor EMSY is described in Hughes-Davies, et al., Cell, 115, 523-535).

Mutations and polymorphisms associated with cancer may be detected at the nucleic acid level by detecting the presence of a variant nucleic acid sequenc or at the protein level by detecting the presence of a variant (i.e. a mutant or allelic variant) polypeptide.

***Definitions***

The fused benzene ring (-A-B)-may bear one or more substituent groups at any available ring position. These substituents are selected from halo, nitro, hydroxy, ether, thiol, thioether, amino, $C_{1-7}$ alkyl, $C_{3-20}$ heterocyclyl and $C_{5-20}$ aryl. The aromatic ring may also bear one or more substituent groups which together form a ring. In particular these may be of formula $-(CH_2)_m-$ or $-O-(CH_2)p-O-$, where m is 2, 3, 4 or 5 and p is 1, 2 or 3.

Alkyl: The term "alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated). Thus, the term "alkyl" includes the sub-classes alkenyl, alkynyl, cycloalkyl, cycloalkyenyl, cylcoalkynyl, etc., discussed below.

In the context of alkyl groups, the prefixes (e.g. $C_{1-4}$, $C_{1-7}$, $C_{1-20}$, $C_{2-7}$, $C_{3-7}$, etc.) denote the number of carbon atoms, or range of number of carbon atoms. For example, the term "$C_{1-4}$ alkyl", as used herein, pertains to an alkyl group having from 1 to 4 carbon atoms. Examples of groups of alkyl groups include $C_{1-4}$ alkyl ("lower alkyl"), $C_{1-7}$ alkyl, and $C_{1-20}$ alkyl. Note that the first prefix may vary according to other limitations; for example, for unsaturated alkyl groups,

the first prefix must be at least 2; for cyclic alkyl groups, the first prefix must be at least 3; etc.

Examples of (unsubstituted) saturated alkyl groups include, but are not limited to, methyl ($C_1$), ethyl ($C_2$), propyl ($C_3$), butyl ($C_4$), pentyl ($C_5$), hexyl ($C_6$), heptyl ($C_7$), octyl ($C_8$), nonyl ($C_9$), decyl ($C_{10}$), undecyl ($C_{11}$), dodecyl ($C_{12}$), tridecyl ($C_{13}$), tetradecyl ($C_{14}$), pentadecyl ($C_{15}$), and eicodecyl ($C_{20}$).

Examples of (unsubstituted) saturated linear alkyl groups include, but are not limited to, methyl ($C_1$), ethyl ($C_2$), n-propyl ($C_3$), n-butyl ($C_4$), n-pentyl (amyl) ($C_5$), n-hexyl ($C_6$), and n-heptyl ($C_7$).

Examples of (unsubstituted) saturated branched alkyl groups include iso-propyl ($C_3$), iso-butyl ($C_4$), sec-butyl ($C_4$), tert-butyl ($C_4$), iso-pentyl ($C_5$), and neo-pentyl ($C_5$).

Alkenyl: The term "alkenyl", as used herein, pertains to an alkyl group having one or more carbon-carbon double bonds. Examples of groups of alkenyl groups include $C_{2-4}$ alkenyl, $C_{2-7}$ alkenyl, $C_{2-20}$ alkenyl.

Examples of (unsubstituted) unsaturated alkenyl groups include, but are not limited to, ethenyl (vinyl, $-CH=CH_2$), 1-propenyl ($-CH=CH-CH_3$), 2-propenyl (allyl, $-CH-CH=CH_2$), isopropenyl (1-methylvinyl, $-C(CH_3)=CH_2$), butenyl ($C_4$), pentenyl ($C_5$), and hexenyl ($C_6$).

Alkynyl: The term "alkynyl", as used herein, pertains to an alkyl group having one or more carbon-carbon triple bonds. Examples of groups of alkynyl groups include $C_{2-4}$ alkynyl, $C_{2-7}$ alkynyl, $C_{2-20}$ alkynyl.

Examples of (unsubstituted) unsaturated alkynyl groups include, but are not limited to, ethynyl (ethinyl, $-C\equiv CH$) and 2-propynyl (propargyl, $-CH_2-C\equiv CH$).

Cycloalkyl: The term "cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms. Thus, the term "cycloalkyl" includes the sub-classes cycloalkenyl and cycloalkynyl. Preferably, each ring has from 3 to 7 ring atoms. Examples of groups of cycloalkyl groups include $C_{3-20}$ cycloalkyl, $C_{3-15}$ cycloalkyl, $C_{3-10}$ cycloalkyl, $C_{3-7}$ cycloalkyl.

Examples of cycloalkyl groups include, but are not limited to, those derived from:

saturated monocyclic hydrocarbon compounds:

cyclopropane ($C_3$), cyclobutane ($C_4$), cyclopentane ($C_5$), cyclohexane ($C_6$), cycloheptane ($C_7$), methylcyclopropane ($C_4$), dimethylcyclopropane ($C_5$), methylcyclobutane ($C_5$), dimethylcyclobutane ($C_6$), methylcyclopentane ($C_6$), dimethylcyclopentane ($C_7$), methylcyclohexane ($C_7$), dimethylcyclohexane ($C_8$), menthane ($C_{10}$);

unsaturated monocyclic hydrocarbon compounds:

cyclopropene ($C_3$), cyclobutene ($C_4$), cyclopentene ($C_5$), cyclohexene ($C_6$), methylcyclopropene ($C_4$), dimethylcyclopropene ($C_5$), methylcyclobutene ($C_5$), dimethylcyclobutene ($C_6$), methylcyclopentene ($C_6$), dimethylcyclopentene ($C_7$), methylcyclohexene ($C_7$), dimethylcyclohexene ($C_8$);

saturated polycyclic hydrocarbon compounds:

thujane ($C_{10}$), carane ($C_{10}$), pinane ($C_{10}$), bornane ($C_{10}$), norcarane ($C_7$), norpinane ($C_7$), norbornane ($C_7$), adamantane ($C_{10}$), decalin (decahydronaphthalene) ($C_{10}$);

unsaturated polycyclic hydrocarbon compounds:

camphene ($C_{10}$), limonene ($C_{10}$), pinene ($C_{10}$);

polycyclic hydrocarbon compounds having an aromatic ring:

indene ($C_9$), indane (e.g., 2,3-dihydro-1H-indene) ($C_9$), tetraline (1,2,3,4-tetrahydronaphthalene) ($C_{10}$), acenaphthene ($C_{12}$), fluorene ($C_{13}$), phenalene ($C_{13}$), acephenanthrene ($C_{15}$), aceanthrene ($C_{16}$), cholanthrene ($C_{20}$).

Heterocyclyl: The term "heterocyclyl", as used herein, pertains to a monovalent moiety obtained by removing a hydrogen atom from a ring atom of a heterocyclic compound, which moiety has from 3 to 20 ring atoms (unless otherwise specified), of which from 1 to 10 are ring heteroatoms. Preferably, each ring has from 3 to 7 ring atoms, of which from 1 to 4 are ring heteroatoms.

In this context, the prefixes (e.g. $C_{3-20}$, $C_{3-7}$, $C_{5-6}$, etc.) denote the number of ring atoms, or range of number

of ring atoms, whether carbon atoms or heteroatoms. For example, the term "$C_{5-6}$heterocyclyl", as used herein, pertains to a heterocyclyl group having 5 or 6 ring atoms. Examples of groups of heterocyclyl groups include $C_{3-20}$ heterocyclyl, $C_{5-20}$ heterocyclyl, $C_{3-15}$ heterocyclyl, $C_{5-15}$ heterocyclyl, $C_{3-12}$ heterocyclyl, $C_{5-12}$ heterocyclyl, $C_{3-10}$ heterocyclyl, $C_{5-10}$ heterocyclyl, $C_{3-7}$ heterocyclyl, $C_{5-7}$ heterocyclyl, and $C_{5-6}$ heterocyclyl.

Examples of monocyclic heterocyclyl groups include, but are not limited to, those derived from:

$N_1$: aziridine ($C_3$), azetidine ($C_4$), pyrrolidine (tetrahydropyrrole) ($C_5$), pyrroline (e.g., 3-pyrroline, 2,5-dihydropyrrole) ($C_5$), 2H-pyrrole or 3H-pyrrole (isopyrrole, isoazole) ($C_5$), piperidine ($C_6$), dihydropyridine ($C_6$), tetrahydropyridine ($C_6$), azepine ($C_7$);

$O_1$: oxirane ($C_3$), oxetane ($C_4$), oxolane (tetrahydrofuran) ($C_5$), oxole (dihydrofuran) ($C_5$), oxane (tetrahydropyran) ($C_6$), dihydropyran ($C_6$), pyran ($C_6$), oxepin ($C_7$);

$S_1$: thiirane ($C_3$), thietane ($C_4$), thiolane (tetrahydrothiophene) ($C_5$), thiane (tetrahydrothiopyran) ($C_6$), thiepane ($C_7$);

$O_2$: dioxolane ($C_5$), dioxane ($C_6$), and dioxepane ($C_7$);

$O_3$: trioxane ($C_6$);

$N_2$: imidazolidine ($C_5$), pyrazolidine (diazolidine) ($C_5$), imidazoline ($C_5$), pyrazoline (dihydropyrazole) ($C_5$), piperazine ($C_6$);

$N_1O_1$: tetrahydrooxazole ($C_5$), dihydrooxazole ($C_5$), tetrahydroisoxazole ($C_5$), dihydroisoxazole ($C_5$), morpholine ($C_6$), tetrahydrooxazine ($C_6$), dihydrooxazine ($C_6$), oxazine ($C_6$);

$N_1S_1$: thiazoline ($C_5$), thiazolidine ($C_5$), thiomorpholine ($C_6$);

$N_2O_1$: oxadiazine ($C_6$);

$O_1S_1$: oxathiole ($C_5$) and oxathiane (thioxane) ($C_6$); and,

$N_1O_1S_1$: oxathiazine ($C_6$) .

Examples of substituted (non-aromatic) monocyclic heterocyclyl groups include those derived from saccharides, in cyclic form, for example, furanoses ($C_5$), such as arabinofuranose, lyxofuranose, ribofuranose, and xylofuranse, and pyranoses ($C_6$), such as allopyranose, altropyranose, glucopyranose, mannopyranose, gulopyranose, idopyranose, galactopyranose, and talopyranose.

Spiro-$C_{3-7}$ cycloalkyl or heterocyclyl: The term "spiro $C_{3-7}$ cycloalkyl or heterocyclyl" as used herein, refers to a $C_{3-7}$ cycloalkyl or $C_{3-7}$ heterocyclyl ring joined to another ring by a single atom common to both rings.

$C_{5-20}$ aryl: The term "$C_{5-20}$ aryl" as used herein, pertains to a monovalent moiety obtained by removing a hydrogen atom from an aromatic ring atom of a $C_{5-20}$ aromatic compound, said compound having one ring, or two or more rings (e.g., fused), and having from 5 to 20 ring atoms, and wherein at least one of said ring(s) is an aromatic ring. Preferably, each ring has from 5 to 7 ring atoms.

The ring atoms may be all carbon atoms, as in "carboaryl groups" in which case the group may conveniently be referred to as a "$C_{5-20}$ carboaryl" group.

Examples of $C_{5-20}$ aryl groups which do not have ring heteroatoms (i.e. $C_{5-20}$ carboaryl groups) include, but are not limited to, those derived from benzene (i.e. phenyl) ($C_6$), naphthalene ($C_{10}$), anthracene ($C_{14}$), phenanthrene ($C_{14}$), and pyrene ($C_{16}$).

Alternatively, the ring atoms may include one or more heteroatoms, including but not limited to oxygen, nitrogen, and sulfur, as in "heteroaryl groups". In this case, the group may conveniently be referred to as a "$C_{5-20}$ heteroaryl" group, wherein "$C_{5-20}$" denotes ring atoms, whether carbon atoms or heteroatoms. Preferably, each ring has from 5 to 7 ring atoms, of which from 0 to 4 are ring heteroatoms.

Examples of $C_{5-20}$ heteroaryl groups include, but are not limited to, $C_5$ heteroaryl groups derived from furan (oxole), thiophene (thiole), pyrrole (azole), imidazole (1,3-diazole), pyrazole (1,2-diazole), triazole, oxazole, isoxazole, thiazole, isothiazole, oxadiazole, tetrazole and oxatriazole; and $C_6$ heteroaryl groups derived from isoxazine, pyridine (azine), pyridazine (1,2-diazine), pyrimidine (1,3-diazine; e.g., cytosine, thymine, uracil), pyrazine (1,4-diazine) and triazine.

The heteroaryl group may be bonded via a carbon or hetero ring atom.

Examples of $C_{5-20}$ heteroaryl groups which comprise fused rings, include, but are not limited to, $C_9$ heteroaryl groups derived from benzofuran, isobenzofuran, benzothiophene, indole, isoindole; $C_{10}$ heteroaryl groups derived from quinoline, isoquinoline, benzodiazine, pyridopyridine; $C_{14}$ heteroaryl groups derived from acridine and xanthene.

The above alkyl, heterocyclyl, and aryl groups, whether alone or part of another substituent, may themselves optionally be substituted with one or more groups selected from themselves and the additional substituents listed below.

Halo: -F, -Cl, -Br, and -I.

Hydroxy: -OH.

Ether: -OR, wherein R is an ether substituent, for example, a $C_{1-7}$ alkyl group (also referred to as a $C_{1-7}$ alkoxy group), a $C_{3-20}$ heterocyclyl group (also referred to as a $C_{3-20}$ heterocyclyloxy group), or a $C_{5-20}$ aryl group (also referred to as a $C_{5-20}$ aryloxy group), preferably a $C_1$-$_7$ alkyl group.

Nitro: $-NO_2$.

Cyano (nitrile, carbonitrile): -CN.

Acyl (keto): -C(=O)R, wherein R is an acyl substituent, for example, H, a $C_{1-7}$ alkyl group (also referred to as $C_{1-7}$ alkylacyl or $C_{1-7}$ alkanoyl), a $C_{3-20}$ heterocyclyl group (also referred to as $C_{3-20}$ heterocyclylacyl), or a $C_{5-20}$ aryl group (also referred to as $C_{5-20}$ arylacyl), preferably a $C_{1-7}$ alkyl group. Examples of acyl groups include, but are not limited to, $-C(=O)CH_3$ (acetyl), $-C(=O)CH_2CH_3$ (propionyl), $-C(=O)C(CH_3)_3$ (butyryl), and -C(=O)Ph (benzoyl, phenone).

Carboxy (carboxylic acid): -COOH.

Ester (carboxylate, carboxylic acid ester, oxycarbonyl): -C(=O)OR, wherein R is an ester substituent, for example, a $C_{1-7}$ alkyl group, a $C_{3-20}$ heterocyclyl group, or a $C_{5-20}$ aryl group, preferably a $C_{1-7}$ alkyl group. Examples of ester groups include, but are not limited to, $-C(=O)OCH_3$, $-C(=O)OCH_2CH_3$, $-C(=O)OC(CH_3)_3$, and -C(=O)OPh.

Amido (carbamoyl, carbamyl, aminocarbonyl, carboxamide): $-C(=O)NR^1R^2$, wherein $R^1$ and $R^2$ are independently amino substituents, as defined for amino groups. Examples of amido groups include, but are not limited to, $-C(=O)NH_2$, $-C(=O)NHCH_3$, $-C(=O)N(CH_3)_2$, $-C(=O)NHCH_2CH_3$, and $-C(=O)N(CH_2CH_3)_2$, as well as amido groups in which $R^1$ and $R^2$, together with the nitrogen atom to which they are attached, form a heterocyclic structure as in, for example, piperidinocarbonyl, morpholinocarbonyl, thiomorpholinocarbonyl, and piperazinylcarbonyl.

Amino: $-NR^1R^2$, wherein $R^1$ and $R^2$ are independently amino substituents, for example, hydrogen, a $C_{1-7}$ alkyl group (also referred to as $C_{1-7}$ alkylamino or di-$C_{1-7}$ alkylamino), a $C_{3-20}$ heterocyclyl group, or a $C_{5-20}$ aryl group, preferably H or a $C_{1-7}$ alkyl group, or, in the case of a "cyclic" amino group, $R^1$ and $R^2$, taken together with the nitrogen atom to which they are attached, form a heterocyclic ring having from 4 to 8 ring atoms. Examples of amino groups include, but are not limited to, $-NH_2$, $-NHCH_3$, $-NHCH(CH_3)_2$, $-N(CH_3)_2$, $-N(CH_2CH_3)_2$, and -NHPh. Examples of cyclic amino groups include, but are not limited to, aziridinyl, azetidinyl, pyrrolidinyl, piperidino, piperazinyl, perhydrodiazepinyl, morpholino, and thiomorpholino. The cylic amino groups may be substituted on their ring by any of the substituents defined here, for example carboxy, carboxylate and amido.

Acylamido (acylamino): $-NR^1C(=O)R^2$, wherein $R^1$ is an amide substituent, for example, hydrogen, a $C_{1-7}$ alkyl group, a $C_{3-20}$ heterocyclyl group, or a $C_{5-20}$ aryl group, preferably H or a $C_{1-7}$ alkyl group, most preferably H, and $R^2$ is an acyl substituent, for example, a $C_{1-7}$ alkyl group, a $C_{3-20}$ heterocyclyl group, or a $C_{5-20}$ aryl group, preferably a $C_{1-7}$ alkyl group. Examples of acylamide groups include, but are not limited to, $-NHC(=O)CH_3$ , $-NHC(=O)CH_2CH_3$, and -NHC (=O)Ph. $R^1$ and $R^2$ may together form a cyclic structure, as in, for example, succinimidyl, maleimidyl, and phthalimidyl:

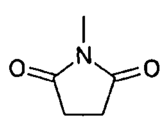
succinimidyl

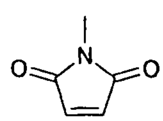
maleimidyl

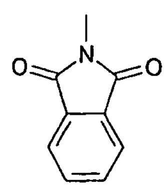
phthalimidyl

Ureido: $-N(R^1)CONR^2R^3$ wherein $R^2$ and $R^3$ are independently amino substituents, as defined for amino groups, and R1 is a ureido substituent, for example, hydrogen, a $C_{1-7}$alkyl group, a $C_{3-20}$heterocyclyl group, or a $C_{5-20}$aryl group, preferably hydrogen or a $C_{1-7}$alkyl group. Examples of ureido groups include, but are not limited to, $-NHCONH_2$, -NH-CONHMe, -NHCONHEt, $-NHCONMe_2$, $-NHCONEt_2$, $NMeCONH_2$, -NMeCONHMe, -NMeCONHEt, $-NMeCONMe_2$, $-NMeCONEt_2$ and - NHC(=O)NHPh.

Acyloxy (reverse ester): -OC(=O)R, wherein R is an acyloxy substituent, for example, a $C_{1-7}$ alkyl group, a $C_{3-20}$ heterocyclyl group, or a $C_{5-20}$ aryl group, preferably a $C_{1-7}$ alkyl group. Examples of acyloxy groups include, but are not limited to, $-OC(=O)CH_3$ (acetoxy), - $OC(=O)CH_2CH_3$, $-OC(=O)C(CH_3)_3$, -OC(=O)Ph, $-OC(=O)C_6H_4F$, and -OC $(=O)CH_2Ph$.

Thiol : -SH.

Thioether (sulfide): -SR, wherein R is a thioether substituent, for example, a $C_{1-7}$ alkyl group (also referred to as a $C_{1-7}$ alkylthio group), a $C_{3-20}$ heterocyclyl group, or a $C_{5-20}$ aryl group, preferably a $C_{1-7}$ alkyl group. Examples of $C_{1-7}$ alkylthio groups include, but are not limited to, $-SCH_3$ and $-SCH_2CH_3$.

Sulfoxide (sulfinyl): -S(=O)R, wherein R is a sulfoxide substituent, for example, a $C_{1-7}$ alkyl group, a $C_{3-20}$ heterocyclyl group, or a $C_{5-20}$ aryl group, preferably a $C_{1-7}$ alkyl group. Examples of sulfoxide groups include, but are not limited to, $-S(=O)CH_3$ and $-S(=O)CH_2CH_3$.

Sulfonyl (sulfone): $-S(=O)_2R$, wherein R is a sulfone substituent, for example, a $C_{1-7}$ alkyl group, a $C_{3-20}$ heterocyclyl group, or a $C_{5-20}$ aryl group, preferably a $C_{1-7}$ alkyl group. Examples of sulfone groups include, but are not limited to, $-S(=O)_2CH_3$ (methanesulfonyl, mesyl), $-S(=O)_2CF_3$, $-S(=O)_2CH_2CH_3$, and 4-methylphenylsulfonyl (tosyl).

Thioamido (thiocarbamyl): $-C(=S)NR^1R^2$, wherein $R^1$ and $R^2$ are independently amino substituents, as defined for amino groups. Examples of amido groups include, but are not limited to, $-C(=S)NH_2$, $-C(=S)NHCH_3$, $-C(=S)N(CH_3)_2$, and $-C(=S)NHCH_2CH_3$.

Sulfonamino: $-NR^1S(=O)_2R$, wherein $R^1$ is an amino substituent, as defined for amino groups, and R is a sulfonamino substituent, for example, a $C_{1-7}$alkyl group, a $C_{3-20}$heterocyclyl group, or a $C_{5-20}$aryl group, preferably a $C_{1-7}$alkyl group. Examples of sulfonamino groups include, but are not limited to, $-NHS(=O)_2CH_3$, $-NHS(=O)_2Ph$ and $-N(CH_3)S(=O)_2C_6H_5$.

As mentioned above, the groups that form the above listed substituent groups, e.g. $C_{1-7}$ alkyl, $C_{3-20}$ heterocyclyl and $C_{5-20}$ aryl, may themselves be substituted. Thus, the above definitions cover substituent groups which are substituted.

Brief Description of Figures

Figure **1** shows clonogenic survival curves of cells exposed to compound (**4**) of the present invention.

Figure 1A shows Brca1 wild type (11CO:■), heterozygous (Cre6:▲) and deficient (Cre10:●) ES cells under continuous exposure to compound **4**. Error bars represent standard errors of the mean.

Figure 1B shows Brca2 wild type (D3:■), heterozygous (Cre6:▲) and deficient (Cre24:o) ES cells under continuous exposure to compound 4. Error bars represent standard errors of the mean.

Figure 2 shows an analysis of the effects of a compound of the invention (4) in another cell line lacking BRCA2 function in comparison to a BRCA2 complemented line. Data shown is clonogenic survival of Brca2 deficient (V-C8:■) and complemented (V-C8 BAC+:△) cells under continuous exposure to compound 4 at varying concentrations.

Further Preferences

The following preferences can apply to each aspect of the present invention, where applicable.

In the present invention, the fused aromatic ring represented by - A-B-is benzene. As described above, this ring may be substituted, but in some embodiments is preferably unsubstituted.

If the fused aromatic ring represented by -A-B- bears a substituent group, it is preferably attached to the atom which itself is attached to the central ring meta- to the carbonyl group. Thus, the preferred place of substitution is shown in the formula below by *:

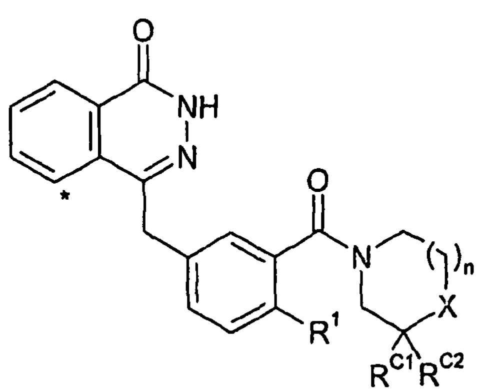

which is usually termed the 5-position of the phthalazinone moiety.

$R^1$ is preferably selected from H, Cl and F, and is more preferably F.

When n is 2, X is NR". In these embodiments, $R^x$ is preferably selected from the group consisting of: H; optionally substituted $C_{1-20}$ alkyl; optionally substituted $C_{5-20}$ aryl; optionally substituted ester groups, wherein the ester substituent is preferably $C_{1-20}$ alkyl; optionally substituted acyl groups; optionally substituted amido groups; optionally substituted thioamido groups; and optionally substituted sulfonyl groups. $R^x$ is more preferably selected from the group consisting of: H; optionally substituted $C_{1-20}$ alkyl; optionally substituted $C_{5-20}$ aryl; and optionally substituted ester groups, wherein the ester substituent is preferably $C_{1-20}$ alkyl.

When n is 1, X may be $NR^X$ or $CR^XCR^Y$.

In embodiments where X is $NR^X$, $R^X$ is preferably selected from the group consisting of: H; optionally substituted $C_{1-20}$ alkyl; optionally substituted $C_{5-20}$ aryl; optionally substituted acyl; optionally substituted sulfonyl; optionally substituted amido; and optionally substituted thioamido groups.

In embodiments where X is $CR^XR^Y$, $R^Y$ is preferably H. $R^X$ is preferably selected from the group consisting of: H; optionally substituted $C_{1-20}$ alkyl; optionally substituted $C_{5-20}$ aryl; optionally substituted $C_{3-20}$ heterocyclyl; optionally substituted acyl, wherein the acyl substituent is preferably selected from $C_{5-20}$ aryl and $C_{3-20}$ heterocylyl (e.g. piperazinyl); optionally substituted amido, wherein the amino groups are preferably selected from H and $C_{1-20}$ alkyl or together with the nitrogen atom, form a $C_{5-20}$ heterocyclic group; and optionally substituted ester groups, wherein the ester substituent is preferably selected from $C_{1-20}$ alkyl groups.

Particularly preferred compounds include: 1, 2, 3, 4, 10, 21, 74, 97, 152, 153, 163, 167, 169, 173, 185, 232, 233, 250, 251, 252, 260 and 263.

Where appropriate, the above preferences may be taken in combination with each other.

Includes Other Forms

Included in the above are the well known ionic, salt, solvate, and protected forms of these substituents. For example, a reference to carboxylic acid (-COOH) also includes the anionic (carboxylate) form ($-COO^-$), a salt or solvate thereof, as well as conventional protected forms. Similarly, a reference to an amino group includes the protonated form ($-N^+HR^1R^2$), a salt or solvate of the amino group, for example, a hydrochloride salt, as well as conventional protected forms of an amino group. Similarly, a reference to a hydroxyl group also includes the anionic form ($-O^-$), a salt or solvate thereof, as well as conventional protected forms of a hydroxyl group.

Isomers, Salts and Solvates

Certain compounds may exist in one or more particular geometric, optical, enantiomeric, diasteriomeric, epimeric, stereoisomeric, tautomeric, conformational, or anomeric forms, including but not limited to, *cis*- and *trans*-forms; *E*- and *Z*-forms; *c*-, *t*-, and *r*-forms; *endo*- and *exo*-forms; *R*-, *S*-, and meso-forms; *D*- and *L*-forms; *d*- and *l*-forms; (+) and (-) forms; keto-, enol-, and enolate-forms; syn- and anti-forms; synclinal- and anticlinal-forms; $\alpha$- and $\beta$-formes; axial and equatorial forms; boat-, chair-, twist-, envelope-, and halfchair-forms; and combinations thereof, hereinafter collectively referred to as "isomers" (or "isomeric forms").

If the compound is in crystalline form, it may exist in a number of different polymorphic forms.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, $-OCH_3$, is not to be construed as a reference to its structural isomer, a hydroxymethyl group, $-CH_2OH$. Similarly, a reference to orthochlorophenyl is not to be construed as a reference to its structural isomer, meta-chlorophenyl. However, a reference to a class of structures may well include structurally isomeric forms falling within that class (e.g., $C_{1-7}$ alkyl includes *n*-propyl and *iso*-propyl; butyl includes *n*-, *iso*-, *sec*-, and *tert*-butyl; methoxyphenyl includes *ortho*-, *meta*-, and *para*-methoxyphenyl).

The above exclusion does not pertain to tautomeric forms, for example, keto-, enol-, and enolate-forms, as in, for example, the following tautomeric pairs: keto/enol, imine/enamine, amide/imino alcohol, amidine/amidine, nitroso/oxime, thioketone/enethiol, *N*-nitroso/hyroxyazo, and nitro/aci-nitro.

Particularly relevant to the present invention is the tautomeric pair illustrated below:

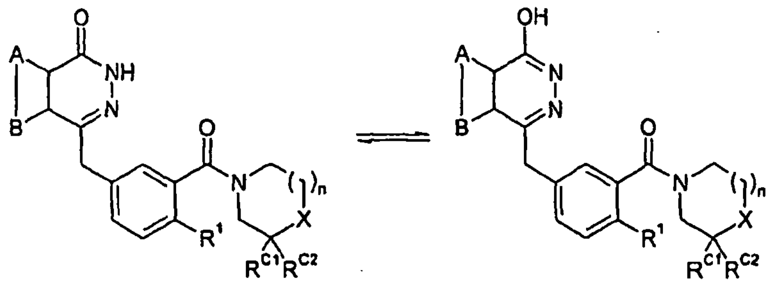

Note that specifically included in the term "isomer" are compounds with one or more isotopic substitutions. For example, H may be in any isotopic form, including $^1H$, $^2H$ (D), and $^3H$ (T); C may be in any isotopic form, including $^{12}C$, $^{13}C$, and $^{14}C$: 0 may be in any isotopic form, including $^{16}O$ and $^{18}O$; and the like.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

Unless otherwise specified, a reference to a particular compound also includes ionic, salt and solvate forms thereof, for example, as discussed below, as well as its different polymorphic forms.

It may be convenient or desirable to prepare, purify, and/or handle a corresponding salt of the active compound, for example, a pharmaceutically-acceptable salt. Examples of pharmaceutically acceptable salts are discussed in Berge, et al., "Pharmaceutically Acceptable Salts", J. Pharm. Sci., 66, 1-19 (1977).

For example, if the compound is anionic, or has a functional group which may be anionic (e.g., -COOH may be $-COO^-$), then a salt may be formed with a suitable cation. Examples of suitable inorganic cations include, but are not limited to, alkali metal ions such as $Na^+$ and $K^+$, alkaline earth cations such as $Ca^{2+}$ and $Mg^{2+}$, and other cations such as $Al^{3+}$. Examples of suitable organic cations include, but are not limited to, ammonium ion (i.e., $NH_4^+$) and substituted ammonium ions (e.g., $NH_3R^+$, $NH_2R_2^+$, $NHR_3^+$, $NR_4^+$). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is $N(CH_3)_4^+$.

If the compound is cationic, or has a functional group which may be cationic (e.g., $-NH_2$ may be $-NH_3^+$), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous. Examples of suitable organic anions include, but are not limited to, those derived from the following organic acids: acetic, propionic, succinic, gycolic, stearic, palmitic, lactic, malic, pamoic, tartaric, citric, gluconic, ascorbic, maleic, hydroxymaleic, phenylacetic, glutamic, aspartic, benzoic, cinnamic, pyruvic, salicyclic, sulfanilic, 2-acetyoxybenzoic, fumaric, toluenesulfonic, methanesulfonic, ethanesulfonic, ethane disulfonic, oxalic, isethionic, valeric, and gluconic. Examples of suitable polymeric anions include, but are not limited to, those derived from the following polymeric acids: tannic acid, carboxymethyl cellulose.

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a tri-hydrate, etc.

Acronyms

For convenience, many chemical moieties are represented using well known abbreviations, including but not limited to, methyl (Me), ethyl (Et), *n*-propyl (nPr), *iso*-propyl (iPr), *n*-butyl (nBu), *tert*-butyl (tBu), *n*-hexyl (nHex), cyclohexyl (cHex), phenyl (Ph), biphenyl (biPh), benzyl (Bn), naphthyl (naph), methoxy (MeO), ethoxy (EtO), benzoyl (Bz), and acetyl (Ac).

For convenience, many chemical compounds are represented using well known abbreviations, including but not limited to, methanol (MeOH), ethanol (EtOH), iso-propanol (*i*-PrOH), methyl ethyl ketone (MEK), ether or diethyl ether ($Et_2O$) acetic acid (AcOH), dichloromethane (methylene chloride, DCM), trifluoroacetic acid (TFA), dimethylformamide (DMF), tetrahydrofuran (THF), and dimethylsulfoxide (DMSO) .

Synthesis

In the synthesis routes given below, the A-B fused ring is shown as an unsubstituted fused benzene ring for convenience. Compounds in which the A-B ring is other than unsubstituted benzene may be synthesised using methodologies analogous to those described below by the use of appropriate alternative starting materials.

Compounds of the present invention may be synthesised by reaction of a compound of Formula 1:

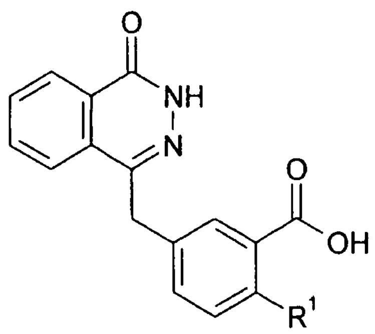

Formula 1

in which $R^1$ is as previously defined, with a compound of Formula 2:

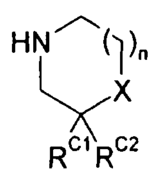

Formula 2

in which n, $R^{C1}$, $R^{C2}$ and X are as previously defined, in the presence of a coupling reagent system, for example **2**-(1*H*-benzotriazol-1-yl)-1,1,3,3-tetramethyluronium tetrafluoroborate, 2-(1*H*-benzotriazol-1-yl)-1,1,3,3-tetramethyluronium hexafluorophosphate or (dimethylaminopropyl)ethylcarbodiimide hydrochloride/hydroxybenzotriazole, in the presence of a base, for example diisopropylethylamine, in a solvent, for example dimethylacetamide or dichloromethane, at a temperature in the range of 0°C to the boiling point of the solvent used.

Alternatively, compounds of the present invention may be synthesised by conversion of a compound of Formula 1 into an activated species, for example an acid chloride or an activated ester such as an *N*-hydroxysuccinimide ester, using well-known methodologies, and reaction of the activated species with a compound of Formula 2.

Compounds of Formula 1 may be synthesised by reaction of a compound of Formula 3:

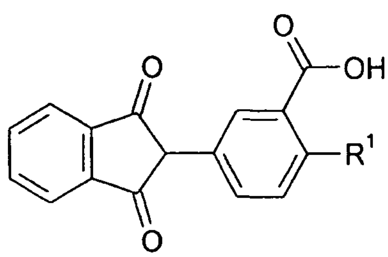

Formula 3

in which $R^1$ is as previously defined, or a compound of Formula 4:

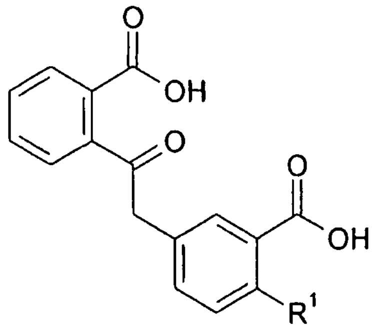

Formula 4

in which $R^1$ is as previously defined, or a mixture of a compound of Formula 3 and a compound of Formula 4, with a source of hydrazine, for example hydrazine hydrate, optionally in the presence of a base, for example triethylamine, optionally in the presence of a solvent, for example industrial methylated spirit, at a temperature in the range of 0˚C to the boiling point of the solvent used.

Compounds of Formula 3 or Formula 4, or mixtures thereof, may be synthesised by reaction of a compound of Formula 5:

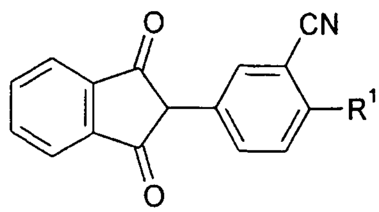

Formula 5

in which $R^1$ is as previously defined, with a reagent capable of hydrolysing a nitrile moiety, for example sodium hydroxide, in the presence of a solvent, for example water, at a temperature in the range of 0˚C to the boiling point of the solvent used.

Compounds of Formula 5 may be synthesised by reaction of a compound of Formula 6:

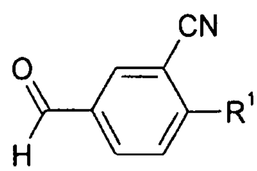

Formula 6

in which $R^1$ is as previously defined, with a compound of Formula 7:

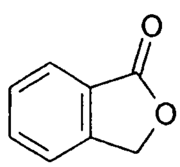

Formula 7

in the presence of a base, for example sodium methoxide, in a solvent, for example methanol, optionally in the presence of a water scavenger, for example ethyl propionate, at a temperature in the range of 0˚C to the boiling point of the solvent used.

Compounds of Formula 1 may also be synthesised by reaction of a compound of Formula 8:

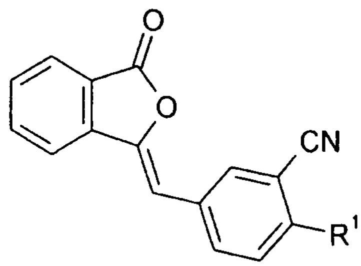

Formula 8

in which $R^1$ is as previously defined, with a reagent capable of hydrolysing a nitrile moiety, for example sodium hydroxide, in the presence of a solvent, for example water, at a temperature in the range of 0˚C to the boiling point of the solvent used, followed by reaction of the resulting intermediate with a source of hydrazine, for example hydrazine hydrate, at a temperature in the range of 0˚C to the boiling point of the solvent used.

Compounds of Formula 8 may be synthesised by reaction of a compound of Formula 9:

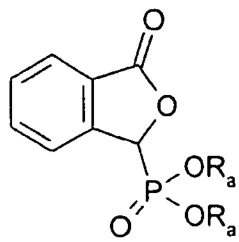

Formula 9

in which $R_a$ is a $C_{1-4}$ alkyl group, with a compound of Formula 6, in the presence of a base, for example triethylamine or lithium hexamethyldisilazide, in the presence of a solvent, for example tetrahydrofuran, at a temperature in the range of -80˚C to the boiling point of the solvent used.

Compounds of Formula 9 may be synthesised by methods analogous to those described in WO 02/26576.

Compounds of Formula 1 may also be synthesised by methods analogous to those described above in which the nitrile moiety in all Formulae is replaced by other moieties capable of generating a carboxylic acid, for example ester or carboxamide moieties.

Compounds of Formula 2 are commercially available or may be synthesised by methods reported in the chemical literature.

Compounds of the present invention in which X is $CR^XR^Y$, in which one of $R^X$ or $R^Y$ is an amido moiety, and which may therefore be represented by Formula 10:

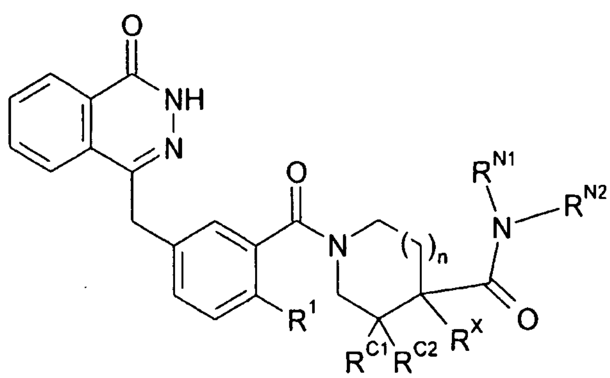

Formula 10

in which n, $R^{C1}$, $R^{C2}$, $R^1$ and $R^X$ are as previously defined and $R^{N1}$ and $R^{N2}$ are each individually selected from the group consisting of H, optionally substituted $C_{1-20}$ alkyl, $C_{5-20}$ aryl, $C_{3-20}$ heterocyclyl, or may together form an optionally substituted $C_{3-7}$ cycloalkyl or heterocyclyl group, may be synthesised by reaction of a compound of Formula 11:

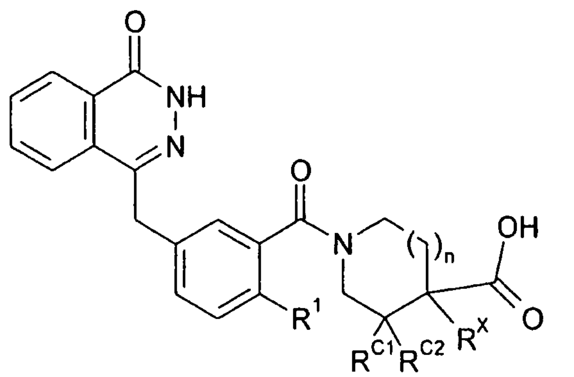

Formula 11

in which n, $R^{C1}$, $R^{C2}$, $R^1$ and $R^N$ are as previously defined, with a compound of Formula $HNR^{N1}R^{N2}$, in which $R^{N1}$ and $R^{N2}$ are as previously defined, in the presence of a coupling reagent system, for example 2-(1*H*-benzotriazol-1-yl)-1,1,3,3-tetramethyluronium tetrafluoroborate, 2-(1*H*-benzotriazol-1-yl)-1,1,3,3-tetramethyluronium hexafluorophosphate or (dimethylaminopropyl)ethylcarbodiimide hydrochloride/ hydroxybenzotriazole, in the presence of a base, for example diisopropylethylamine, in a solvent, for example dimethylacetamide or dichloromethane, at a temperature in the range of 0°C to the boiling point of the solvent used.

Alternatively, compounds of Formula 10 may be synthesised by conversion of a compound of Formula 11 into an activated species, for example an acid chloride or an activated ester such as an *N*-hydroxysuccinimide ester, using well-known methodologies, and reaction of the activated species with a compound of Formula $HNR^{N1}R^{N2}$.

Compounds of Formula 11 may be synthesised by deprotection of a protected form of a compound of Formula 11, for example a compound of Formula 12:

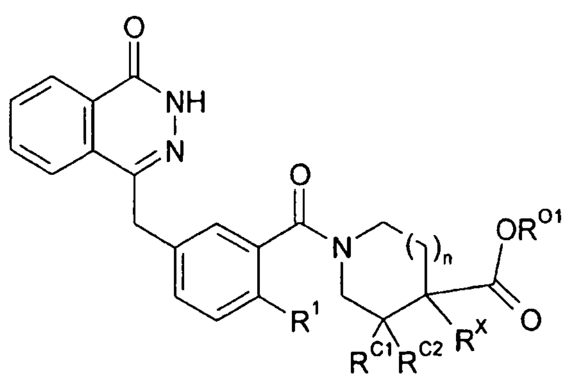

Formula 12

in which n, $R^{C1}$, $R^{C2}$, $R^1$ and $R^X$ are as previously defined and $R^{01}$ is a $C_{1-4}$ alkyl group, using well known methodologies, for example base-catalysed hydrolysis in the presence of a source of hydroxide, for example sodium or lithium hydroxide, in the presence of a solvent, for example water and/or tetrahydrofuran, at a temperature in the range of 0°C to the boiling point of the solvent used.

Compounds of Formula 12 may be synthesised from compounds of Formula 1 by the previously described methods.

Compounds of Formula $HNR^{N1}R^{N2}$ are commercially available or may be synthesised by methods reported in the chemical literature.

Compounds of the present invention in which X is NH and which may therefore be represented by Formula 13:

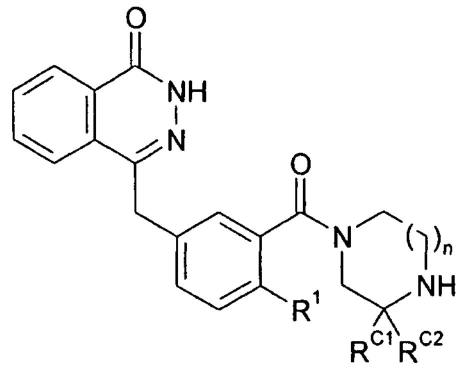

Formula 13

in which n, $R^{C1}$, $R^{C2}$ and $R^1$ are as previously defined, may be synthesised by deprotection of a protected form of a compound of Formula 13, for example a compound of Formula 14:

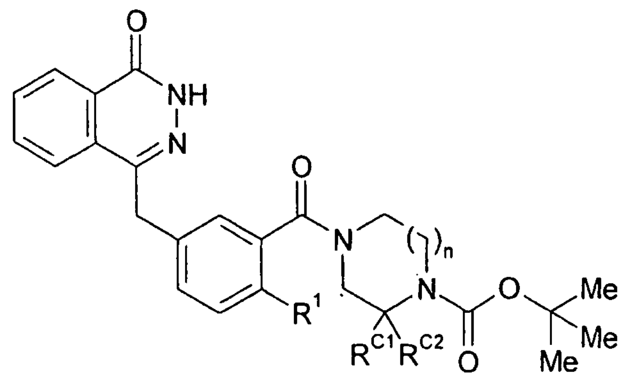

Formula 14

in which n, $R^{C1}$, $R^{C2}$ and $R^1$ are as previously defined, using well known methodologies, for example acid-catalysed cleavage, in the presence of an acid, for example trifluoroacetic acid or hydrochloric acid, in the presence of a solvent, for example dichloromethane or ethanol and/or water, at a temperature in the range of 0°C to the boiling point of the solvent used.

Compounds of Formula 14 may be synthesised from compounds of Formula 1 by the previously described methods.

Compounds of the present invention in which X is $NR^X$, in which $R^X$ is an acyl moiety, and which may therefore be represented by Formula 15:

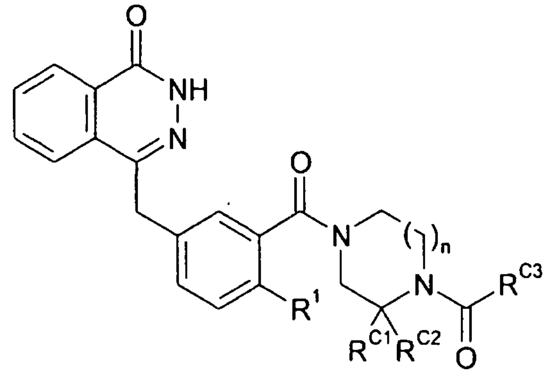

Formula 15

in which n, $R^{C1}$, $R^{C2}$ and $R^1$ are as previously defined and $R^{C3}$ is selected from the group consisting of optionally substituted $C_{1-20}$ alkyl, $C_{5-20}$ aryl and $C_{3-20}$ heterocyclyl, may be synthesised by reaction of a compound of Formula 13 with a compound of Formula $R^{C3}COX$, in which $R^{C3}$ is as previously defined and X is a suitable leaving group, for example a halogen such as chloro, optionally in the presence of a base, for example pyridine, triethylamine or diisopropylethylamine, optionally in the presence of a solvent, for example dichloromethane, at a temperature in the range of 0˚C to the boiling point of the solvent used.

Compounds of Formula $R^{C3}COX$ are commercially available or may be synthesised by methods reported in the chemical literature.

Compounds of Formula 15 may also be synthesised by reaction of a compound of Formula 13 with a compound of Formula $R^{C3}CO_2H$, in which $R^{C3}$ is as previously defined, in the presence of a coupling reagent system, for example 2-(1*H*-benzotriazol-1-yl)-1,1,3,3-tetramethyluronium tetrafluoroborate, 2-(1*H*-benzotriazol-1-yl)-1,1,3,3-tetramethyluronium hexafluorophosphate or (dimethylaminopropyl)ethylcarbodiimide hydrochloride/ hydroxybenzotriazole, in the presence of a base, for example diisopropylethylamine, in a solvent, for example dimethylacetamide or dichloromethane, at a temperature in the range of 0˚C to the boiling point of the solvent used.

Compounds of Formula $R^{C3}CO_2H$ are commercially available or may be synthesised by methods reported in the chemical literature.

Compounds of the present invention in which X is $NR^X$, in which $R^X$ is an amido or thioamido moiety, and which may therefore be represented by Formula 16:

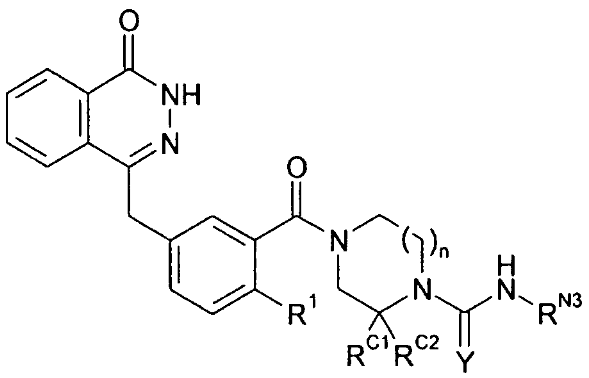

Formula 16

in which n, $R^C$, $R^{C2}$ and $R^1$ are as previously defined, Y is O or S and $R^{N3}$ is selected from the group consisting of optionally substituted $C_{1-20}$ alkyl, $C_{5-20}$ aryl and $C_{3-20}$ heterocyclyl, may be synthesised by reaction of a compound of Formula 13 with a compound of Formula $R^{N3}NCY$, in which Y and $R^{N3}$ are as previously defined, in the presence of a solvent, for example dichloromethane, at a temperature in the range of 0˚C to the boiling point of the solvent used.

Compounds of Formula $R^{N3}NCY$ are commercially available or may be synthesised by methods reported in the chemical literature.

Compounds of the present invention in which X is $NR^X$, in which $R^X$ is a sulfonyl moiety, and which may therefore be represented by Formula 17:

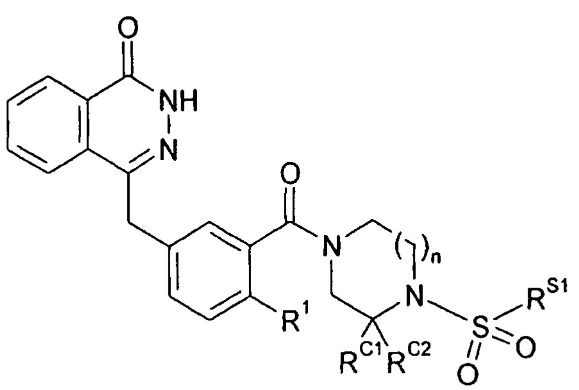

Formula 17

in which n, $R^{C1}$, $R^{C2}$ and $R^1$ are as previously defined and $R^{S1}$ is selected from the group consisting of optionally substituted $C_{1-20}$ alkyl, $C_{5-20}$ aryl and $C_{3-20}$ heterocyclyl, may be synthesised by reaction of a compound of Formula 13 with a compound of Formula $R^{S1}SO_2Cl$, in which $R^{S1}$ is as previously defined, optionally in the presence of a base, for example pyridine, triethylamine or diisopropylethylamine, in the presence of a solvent, for example dichloromethane, at a temperature in the range of 0˚C to the boiling point of the solvent used.

Compounds of Formula $R^{S1}SO_2Cl$ are commercially available or may be synthesised by methods reported in the chemical literature.

Compounds of the present invention in which X is $NR^X$, in which $R^X$ is selected from the group consisting of optionally substituted $C_{1-20}$ alkyl or $C_{3-20}$ heterocyclyl, and which may therefore be represented by Formula 18:

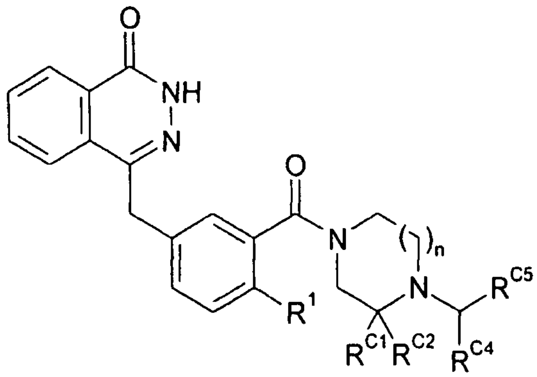

Formula 18

in which n, $R^{C1}$, $R^{C2}$ and $R^1$ are as previously defined and $R^{C4}$ and $R^{C5}$ are each individually selected from the group consisting of H, optionally substituted $C_{1-20}$ alkyl, $C_{5-20}$ aryl, $C_{3-20}$ heterocyclyl, or may together form an optionally substituted $C_{3-7}$ cycloalkyl or heterocyclyl group, may be synthesised by reaction of a compound of Formula 13 with a compound of Formula $R^{C4}COR^{C5}$, in which $R^{C4}$ and $R^{C5}$ are as previously defined, in the presence of a reducing agent, for example sodium cyanoborohydride or sodium triacetoxyborohydride, in the presence of a solvent, for example methanol, optionally in the presence of an acid catalyst, for example acetic acid, at a temperature in the range of 0˚C to the boiling point of the solvent used.

Compounds of Formula $R^{C4}COR^{C5}$ are commercially available or may be synthesised by methods reported in the chemical literature.

Use

The present invention provides active compounds, specifically, active in inhibiting the activity of PARP.

The term "active" as used herein, pertains to compounds which are capable of inhibiting PARP activity, and specifically includes both compounds with intrinsic activity (drugs) as well as prodrugs of such compounds, which prodrugs may themselves exhibit little or no intrinsic activity.

One assay which may conveniently be used in order to assess the PARP inhibition offered by a particular compound is described in the examples below.

The present invention further provides a method of inhibiting the activity of PARP in a cell, comprising contacting said cell with an effective amount of an active compound, preferably in the form of a pharmaceutically acceptable composition. Such a method may be practised *in vitro* or *in vivo.*

For example, a sample of cells may be grown *in vitro* and an active compound brought into contact with said cells, and the effect of the compound on those cells observed. As examples of "effect", the amount of DNA repair effected in a certain time may be determined. Where the active compound is found to exert an influence on the cells, this may be used as a prognostic or diagnostic marker of the efficacy of the compound in methods of treating a patient carrying cells of the same cellular type.

The term "treatment", as used herein in the context of treating a condition, pertains generally to treatment and therapy, whether of a human or an animal (e.g. in veterinary applications), in which some desired therapeutic effect is achieved, for example, the inhibition of the progress of the condition, and includes a reduction in the rate of progress, a halt in the rate of progress, amelioration of the condition, and cure of the condition. Treatment as a prophylactic measure (i.e. prophylaxis) is also included.

The term "adjunct" as used herein relates to the use of active compounds in conjunction with known therapeutic means. Such means include cytotoxic regimes of drugs and/or ionising radiation as used in the treatment of different cancer types. In particular, the active compounds are known to potentiate the actions of a number of cancer chemotherapy treatments, which include the topoisomerase class of poisons (e.g. topotecan, irinotecan, rubitecan), most of the known alkylating agents (e.g. DTIC, temozolamide) and platinum based drugs (e.g. carboplatin, cisplatin) used in treating cancer.

Active compounds may also be used as cell culture additives to inhibit PARP, for example, in order to sensitize cells to known chemotherapeutic agents or ionising radiation treatments *in vitro.*

Active compounds may also be used as part of an *in vitro* assay, for example, in order to determine whether

a candidate host is likely to benefit from treatment with the compound in question.

Administration

The active compound or pharmaceutical composition comprising the active compound may be administered to a subject by any convenient route of administration, whether systemically/ peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot, for example, subcutaneously or intramuscularly.

The subject may be a eukaryote, an animal, a vertebrate animal, a mammal, a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orangutang, gibbon), or a human.

Formulations

While it is possible for the active compound to be administered alone, it is preferable to present it as a pharmaceutical composition (e.g., formulation) comprising at least one active compound, as defined above, together with one or more pharmaceutically acceptable carriers, adjuvants, excipients, diluents, fillers, buffers, stabilisers, preservatives, lubricants, or other materials well known to those skilled in the art and optionally other therapeutic or prophylactic agents.

Thus, the present invention further provides pharmaceutical compositions, as defined above, and methods of making a pharmaceutical composition comprising admixing at least one active compound, as defined above, together with one or more pharmaceutically acceptable carriers, excipients, buffers, adjuvants, stabilisers, or other materials, as described herein.

The term "pharmaceutically acceptable" as used herein pertains to compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable carriers, diluents, excipients, etc. can be found in standard pharmaceutical texts. See, for example, "Handbook of Pharmaceutical Additives", 2nd Edition (eds. M. Ash and I. Ash), 2001 (Synapse Information Resources, Inc., Endicott, New York, USA), "Remington's Pharmaceutical Sciences", 20th edition, pub. Lippincott, Williams & Wilkins, 2000; and "Handbook of Pharmaceutical Excipients", 2nd edition, 1994.

The formulations may conveniently be presented in unit dosage form and may be prepared by any methods well known in the art of pharmacy. Such methods include the step of bringing into association the active compound with the carrier which constitutes one or more accessory ingredients. In general, the formulations are prepared by uniformly and intimately bringing into association the active compound with liquid carriers or finely divided solid carriers or both, and then if necessary shaping the product.

Formulations may be in the form of liquids, solutions, suspensions, emulsions, elixirs, syrups, tablets, losenges, granules, powders, capsules, cachets, pills, ampoules, suppositories, pessaries, ointments, gels, pastes, creams, sprays, mists, foams, lotions, oils, boluses, electuaries, or aerosols.

Formulations suitable for oral administration (e.g., by ingestion) may be presented as discrete units such as capsules, cachets or tablets, each containing a predetermined amount of the active compound; as a powder or granules; as a solution or suspension in an aqueous or non-aqueous liquid; or as an oil-in-water liquid emulsion or a water-in-oil liquid emulsion; as a bolus; as an electuary; or as a paste.

A tablet may be made by conventional means, e.g. compression or molding, optionally with one or more accessory ingredients. Compressed tablets may be prepared by compressing in a suitable machine the active compound in a free-flowing form such as a powder or granules, optionally mixed with one or more binders (e.g. povidone, gelatin, acacia, sorbitol, tragacanth, hydroxypropylmethyl cellulose); fillers or diluents (e.g. lactose, microcrystalline cellulose, calcium hydrogen phosphate); lubricants (e.g. magnesium stearate, talc, silica); disintegrants (e.g. sodium starch glycolate, cross-linked povidone, cross-linked sodium carboxymethyl cellulose); surface-active or dispersing or wetting agents (e.g., sodium lauryl sulfate); and preservatives (e.g., methyl p-hydroxybenzoate, propyl p-hydroxybenzoate, sorbic acid). Molded tablets may be made by molding in a suitable machine a mixture of the powdered compound moistened with an inert liquid diluent. The tablets may optionally be coated or scored and may be formulated so as to

provide slow or controlled release of the active compound therein using, for example, hydroxypropylmethyl cellulose in varying proportions to provide the desired release profile. Tablets may optionally be provided with an enteric coating, to provide release in parts of the gut other than the stomach.

Formulations suitable for topical administration (e.g. transdermal, intranasal, ocular, buccal, and sublingual) may be formulated as an ointment, cream, suspension, lotion, powder, solution, past, gel, spray, aerosol, or oil. Alternatively, a formulation may comprise a patch or a dressing such as a bandage or adhesive plaster impregnated with active compounds and optionally one or more excipients or diluents.

Formulations suitable for topical administration in the mouth include losenges comprising the active compound in a flavored basis, usually sucrose and acacia or tragacanth; pastilles comprising the active compound in an inert basis such as gelatin and glycerin, or sucrose and acacia; and mouthwashes comprising the active compound in a suitable liquid carrier.

Formulations suitable for topical administration to the eye also include eye drops wherein the active compound is dissolved or suspended in a suitable carrier, especially an aqueous solvent for the active compound.

Formulations suitable for nasal administration, wherein the carrier is a solid, include a coarse powder having a particle size, for example, in the range of about 20 to about 500 microns which is administered in the manner in which snuff is taken, i.e. by rapid inhalation through the nasal passage from a container of the powder held close up to the nose. Suitable formulations wherein the carrier is a liquid for administration as, for example, nasal spray, nasal drops, or by aerosol administration by nebuliser, include aqueous or oily solutions of the active compound.

Formulations suitable for administration by inhalation include those presented as an aerosol spray from a pressurised pack, with the use of a suitable propellant, such as dichlorodifluoromethane, trichlorofluoromethane, dichorotetrafluoroethane, carbon dioxide, or other suitable gases.

Formulations suitable for topical administration via the skin include ointments, creams, and emulsions. When formulated in an ointment, the active compound may optionally be employed with either a paraffinic or a water-miscible ointment base. Alternatively, the active compounds may be formulated in a cream with an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues.

When formulated as a topical emulsion, the oily phase may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulphate. The choice of suitable oils or fats for the formulation is based on achieving the desired cosmetic properties, since the solubility of the active compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active compound, such carriers as are known in the art to be appropriate.

Formulations suitable for parenteral administration (e.g., by injection, including cutaneous, subcutaneous, intramuscular, intravenous and intradermal), include aqueous and non-aqueous isotonic, pyrogen-free, sterile injection solutions which may contain anti-oxidants, buffers, preservatives, stabilisers, bacteriostats, and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer=s Solution, or Lactated Ringer=$_s$ Injection. Typically, the concentration of the active compound in the solution is from about 1 ng/ml to about 10 μg/ml, for example

from about 10 ng/ml to about 1 μg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to blood components or one or more organs.

Dosage

It will be appreciated that appropriate dosages of the active compounds, and compositions comprising the active compounds, can vary from patient to patient. Determining the optimal dosage will generally involve the balancing of the level of therapeutic benefit against any risk or deleterious side effects of the treatments of the present invention. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

Administration *in vivo* can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound is in the range of about 100 μg to about 250 mg per kilogram body weight of the subject per day. Where the active compound is a salt, an ester, prodrug, or the like, the amount administered is calculated on the basis of the parent compound and so the actual weight to be used is increased proportionately.

Synthesis Data

General Experimental Methods

**Preparative HPLC**

Samples were purified with a Waters mass-directed purification system utilising a Waters 600 LC pump, Waters Xterra C18 column (5 μm 19 mm x 50 mm) and Micromass ZQ mass spectrometer, operating in positive ion electrospray ionisation mode. Mobile phases A (0.1% formic acid in water) and B (0.1 % formic acid in acetonitrile) were used in a gradient; 5% B to 100% over 7 min, held for 3 min, at a flow rate of 20 ml/ min.

**Analytical HPLC-MS**

Analytical HPLC was carried out with a Spectra System P4000 pump and Jones Genesis C18 column (4 μm, 50 mm x 4.6 mm). Mobile phases A (0.1 % formic acid in water) and B (acetonitrile) were used in a gradient of 5 % B for 1 min rising to 98 % B after 5 min, held for 3 min at a flow rate of 2 ml / min. Detection was by a TSP UV 6000LP detector at 254 nm UV and range 210-600 nm PDA. The Mass spectrometer was a Finnigan LCQ operating in positive ion electrospray mode.

**NMR**

$^{1}H$ NMR and $^{13}C$ NMR were recorded using Bruker DPX 300 spectrometer at 300 MHz and 75 MHz respectively. Chemical shifts were reported in parts per million (ppm) on the δ scale relative to tetramethylsilane internal standard. Unless stated otherwise all samples were dissolved in DMSO-$d_6$.

***Synthesis of Key Intermediates***

*a. 3-(4-0xo-3,4-dihydrophthalazin-1-ylmethyl)benzoic acid (A)*

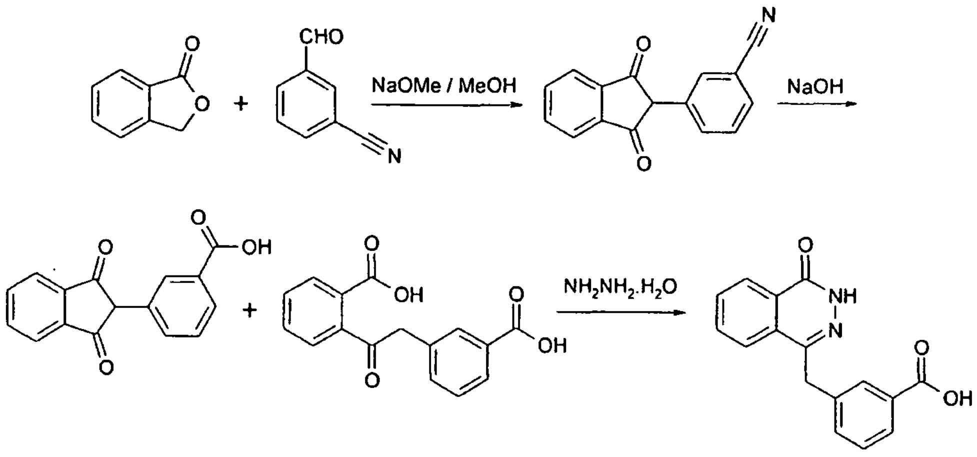


(A)

A mixture of 27% sodium methoxide solution in methanol (400 g, 2 mol) and methanol (150 ml) was added dropwise between ambient temperature and 30˚C over 15 minutes to a stirred mixture of phthalide (67 g, 0.5 mol), 3-formylbenzonitrile (65.5 g, 0.5 mol) and ethyl propionate (250 ml), the mixture was stirred at ambient temperature for 40 minutes and at reflux temperature for 1 hour, then it was allowed to cool to ambient temperature. The resulting red solid was collected by filtration, washed with ethyl acetate (2 x 50 ml) and dissolved in water (1800 ml). The solution was acidified by the addition of acetic acid (60 ml) and the resulting red solid was collected by filtration, washed with water (2 x 200 ml) and dried *in vacuo* to give 3-(1,3-dioxoindan-2-yl)benzonitrile (83.2 g) as a dark red solid, m.pt. 179-182˚C, m/z $(M+H)^{+\cdot}$ 248, which was used without further purification.

3-(1,3-Dioxoindan-2-yl)benzonitrile (74.18 g, 0.3 mol) was added in portions to a solution of sodium hydroxide (36 g, 0.9 mol) in water (580 ml), the resulting dark red suspension was stirred at reflux temperature for 5 hours, then it was cooled to ambient temperature and washed with ethyl acetate (3 x 300 ml). The aqueous solution was acidified by the dropwise addition of concentrated hydrochloric acid (110 ml), the mixture was stirred at ambient temperature for 1 hour, then the resulting solid was collected by filtration, washed with water (2 x 200 ml) and dried *in vacuo* to give a 1:1 mixture of 3-(1,3-dioxoindan-2-yl)benzoic acid, $(M+H)^{+\cdot}$ 267, and 2-[2-(3-carboxyphenyl)acetyl]benzoic acid, $(M+H)^{+\cdot}$ 285, (69.32 g), which was used without further purification.

The mixture obtained in the previous step (52.8 g) was added to a solution of triethylamine (37.55 g, 0.372 mol) in industrial methylated spirit (500 ml) and the resulting cloudy solution was filtered through a pad of filter-aid to give a clear solution. Hydrazine monohydrate (9.3 g, 0.186 mol) was added in one portion at ambient temperature, the stirred mixture was heated under reflux for 1 hour, then it was concentrated *in vacuo* to approximately 250 ml and added to a solution of sodium acetate (41 g, 0.5 mol) in water (500 ml). The mixture was brought to pH 7 by the dropwise addition of concentrated hydrochloric acid, then it was stirred at ambient temperature for 3 hours. The resulting solid was collected by filtration, washed with water (50 ml) and dried *in vacuo* to give a white solid (15.62 g). The combined filtrate and washings were acidified to pH 6 by the addition of hydrochloric acid, then the mixture was stirred at ambient temperature for 3 hours. The resulting solid was collected by filtration, washed with water (50 ml) and dried *in vacuo* to give a second crop of off-white solid (17.57 g). The combined filtrate and washings from the second crop were readjusted to pH 6 and treated as before to give a third crop of pale orange solid (6.66 g). The three crops were combined to give essentially pure 3-(4-oxo-3,4-dihydrophthalazin-1-ylmethyl)benzoic acid (A), $(M+H)^{+\cdot}$ 281, $\delta_H$ 4.4 (2H, s), 7.2-7.4 (1H, m), 7.5-7.6 (1H, m), 7.7-8.0 (5H, m), 8.1-8.2 (1H, m), 12.6 (1H, s)

b. *2-Fluoro-5-(4-oxo-3,4-dihydro-phthalazin-1-ylmethyl)benzoic acid (B)*

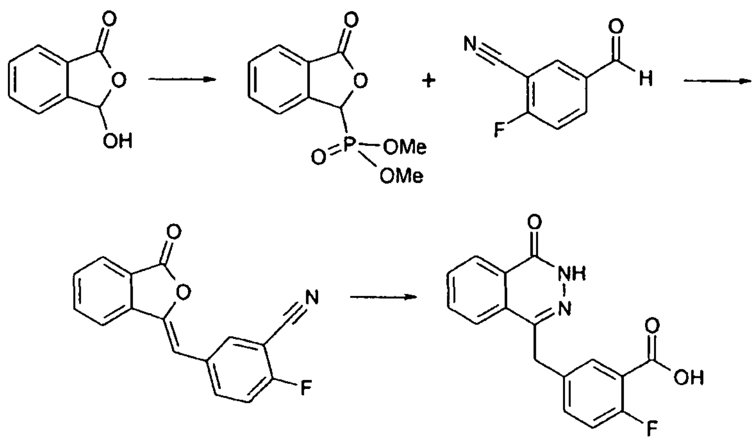

(B)

Dimethyl phosphite (22.0 g, 0.2 mol) was added drop-wise to a solution of sodium methoxide (43.0 g) in methanol (100 ml) at 0˚C. 2-Carboxybenzaldehyde (21.0 g, 0.1 mol) was then added portion-wise to the reaction mixture as a slurry in methanol (40 ml), with the temperature kept below 5˚C. The resulting pale yellow solution was warmed to 20˚C over 1 hour. Methanesulphonic acid (21.2 g, 0.22 mol) was added to the reaction drop-wise and the resulting white suspension was evaporated *in vacuo.* The white residue was quenched with water and extracted into chloroform (3 x 100 ml). The combined organic extracts were washed with water (2 x 100 ml), dried over $MgSO_4$ and evaporated *in vacuo* to yield (3-oxo-1,3-dihydro-isobenzofuran-1-yl)phosphonic acid dimethyl ester as a white solid (32.0 g, 95 %, 95 % purity). This was then used without further purification in the next stage.

To a mixture of (3-oxo-1,3-dihydro-isobenzofuran-1-yl)phosphonic acid dimethyl ester (35.0 g, 0.14 mol) in tetrahydrofuran (200 ml) and 2-fluoro-5-formylbenzonitrile (20.9 g, 0.14 mol) in tetrahydrofuran (130 ml) was added triethylamine (14 ml, 0.14 mol) drop-wise over 25 min, with the temperature kept below 15˚C. The reaction mixture was warmed slowly to 20˚C over 1 hour and concentrated *in vacuo.* The white residue was slurried in water (250 ml) for 30 minutes, filtered, washed with water, hexane and ether, and dried to yield 2-fluoro-5-(3-oxo-3H-isobenzofuran-1-ylidenemethyl)benzonitrile as a 50:50 mixture of E and Z isomers (37.2 g, 96 %); m/z $[M+1]^+$ 266 (98 % purity)

To a suspension of 2-fluoro-5-(3-oxo-3H-isobenzofuran-1-ylidenemethyl)benzonitrile in water (200 ml) was added aqueous sodium hydroxide (26.1 g in 50 ml water) solution and the reaction mixture was heated under nitrogen to 90˚C for 30 minutes. The reaction mixture was partially cooled to 70˚C, and hydrazine hydrate (100 ml) was added and stirred for 18 hours at 70˚C. The reaction was cooled to room temperature and acidified with 2M HCl to pH 4. The mixture was stirred for 10 min and filtered. The resulting solid was washed with water, hexane, ether, ethyl acetate and dried to yield 2-fluoro-5-(4-oxo-3,4-dihydrophthalazin-1-ylmethyl)benzoic acid as a pale pink powder (30.0 g, 77 %). m/z $[M+1]^+$ 299 (96 % purity), $\delta_H$ 4.4 (2H, s), 7.2-7.3 (1H, m), 7.5-7.6 (1H, m), 7.8-8.0 (4H, m), 8.2-8.3 (1H, m), 12.6 (1H, s).

c. *1-(3-(4-Oxo-3,4-dihydrophthalazin-1-ylmethyl)benzoyl)piperidine-4-carboxylic acid (C)*

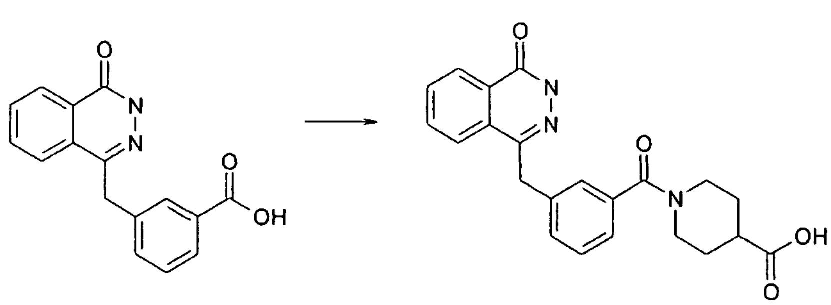

(A) (C)

3-(4-Oxo-3,4-dihydrophthalazin-1-ylmethyl)benzoic acid (A)(7.0 g, 0.25 mol), ethyl isonipecotate (5 ml, 0.32 mol), 2-(1H-benzotriazol-1-yl)-1,1,3,3-tetramethyluronium hexafluorophosphate (HBTU) (12.3 g, 0.32 mol) and N,N,-diisopropylethylamine (10.0 ml, 0.55 mol) were added to dimethylacetamide (40 ml) and stirred for 18 h. Water (100 ml) was added to the reaction mixture and the product was extracted into dichloromethane (4 x 50 ml). The combined organic layers were washed with water (3 x 100 ml), dried over $MgSO_4$, filtered and evaporated *in vacuo* to yield an oil. To a solution of the oil in tetrahydrofuran (100 ml) was added 10 % aqueous sodium hydroxide solution(20 ml) and the reaction was stirred for 18 hours. The reaction was concentrated, washed with ethyl acetate (2 x 30 ml) and acidified with 2M HCl to pH 2. The aqueous layer was extracted with dichloromethane (2 x 100 ml), then the extracts were dried over $MgSO_4$, filtered and evaporated to yield 1-[3-(4-oxo-3,4-dihydrophthalazin-1-ylmethyl)benzoyl]piperidine-4-carboxylic acid (C) as a yellow solid (7.0 g, 65 z), m/z $[M+1]^+$ 392 (96 % purity), $\delta_H$ 1.3-1.8 (5H, m), 2.8-3.1 (4H, m), 4.4 (2H, s), 7.2-7.3 (1H, m), 7.3-7.4 (1H, m), 7.7-8.0 (5H, m), 8.2-8.3 (1H, m), 12.6 (1H, s).

*d. 1-[2-Fluoro-5-(9-oxo-3,9-dihydrophthalazin-1-ylmethyl)benzoyl]piperidine-4-carboxylic acid (D)*

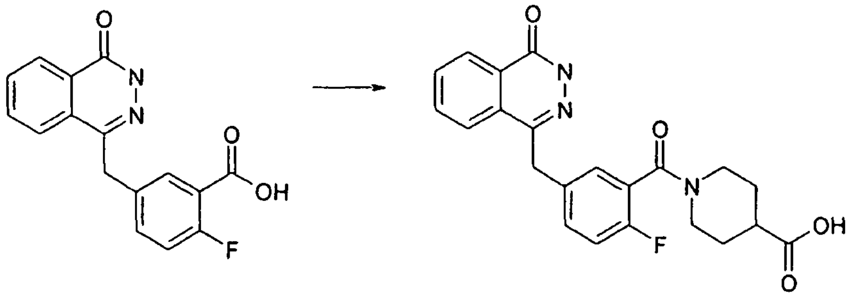

(B) (D)

2-Fluoro-5-(4-oxo-3,9-dihydrophthalazin-1-ylmethyl)benzoic acid (B)(3.1 g, 0.14 mol), ethyl isonipecotate (1.7 ml, 0.11 mol), 2-(1H-benzotriazol-1-yl)-1,1,3,3-tetramethyluronium hexafluorophosphate (HBTU) (5.1 g, 0.13 mol) and N,N,-diisopropylethylamine (10.0 ml, 0.55 mol) were added to dimethylacetamide (15 ml) and stirred for 18 hours. Water (100 ml) was added to the reaction mixture and the product was extracted into dichloromethane (4 x 50 ml). The combined organic layers were, filtered, washed with water (3 x 100 ml), dried over $MgSO_4$, filtered and evaporated *in vacuo* to yield an orange oil. The oil was purified by flash chromatography (ethyl acetate) to yield 1-[2-fluoro-5-(4-oxo-3,4-dihydrophthalazin-1-ylmethyl)benzoyl]piperidine-4-carboxylic acid as the methyl ester (1.5 g, 33 %, 96 % purity). To a solution of the methyl ester in tetrahydrofuran: water (2:1, 40 ml) was added sodium hydroxide (0.3 g, 0.075 mol) and the reaction was stirred for 18 h. The reaction was concentrated, washed with ethyl acetate (2 x 20 ml) and acidified with 2M HCl to pH 2. The aqueous layer was extracted with dichloromethane (2 x 20 ml), and the combined extracts were dried over $MgSO_4$ and evaporated to yield 1-[3-(4-oxo-3,4-dihydrophthalazin-1-ylmethyl)benzoyllpiperidine-4-carboxylic acid (D) as a yellow solid (0.6 g, 65 %), m/z $[M+1]^+$ 392 (96 % purity)

**Example 1 - Synthesis of Key Compounds**

**a. Synthesis of 4-[3-(piperazine-1-carbonyl)benzyl]-2H-phthalazin-1-one (1)**

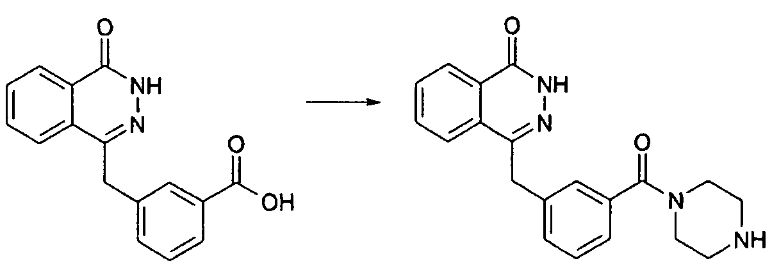

(A) (1)

3-(4-Oxo-3,4-dihydrophthalazin-1-ylmethyl)benzoic acid (A) (5.0g, 0.17mol)), tert-butyl 1-piperazinecarboxylate (3.9 g, 0.21 mol), 2-(1H-benzotriazol-1-yl)-1,1,3,3-tetramethyluronium hexafluorophosphate (HBTU) (8.6 g, 0.22 mol) and N,N,-diisopropylethylamine (6.7 ml, 0.38 mol) were added to dimethylacetamide (40 ml) and stirred for 18 hours. Water (100 ml) was added and the reaction mixture was heated to 100˚C for 1 hour. The suspension was cooled to room temperature, filtered and dried to yield a white solid. The solid was dissolved in a solution of 6M HCl and ethanol (2:1, 50 ml) and stirred for 1 hour. The reaction was concentrated, basified with ammonia to pH 9, and the product was extracted into dichloromethane (2 x 50 ml). The combined organic layers were washed with water (2 x 50 ml), dried over $MgSO_4$, and evaporated *in vacuo* to yield 4-[3-(piperazine-1-carbonyl)benzyl]-2H-phthalazin-1-one (1) as a yellow crystalline solid (4.0 g, 77 %); m/z $[M+1]^+$ 349 (97 % purity), $\delta_H$ 2.6-3.8 (8H, m), 4.4 (2H, s), 7.2-7.5 (4H, m), 7.7-8.0 (3H, m), 8.2-8.3 (1H, m), 12.6 (1H, s)

*b. Synthesis of 4-[4-Fluoro-3-(piperazine-1-carbonyl)benzyl]-2H-phthalazin-1-one (2)*

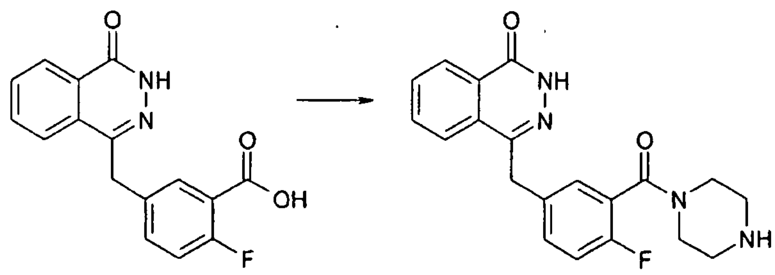

(B) (2)

The synthesis was carried out according to the method described in (a) above using 2-fluoro-5-(4-oxo-3,4-dihydrophthalazin-1-ylmethyl)benzoic acid (B) to yield 9-[9-fluoro-3-(piperazine-1-carbonyl)benzyl]-2H-phthalazin-1-one (2) as a white crystalline solid (4.8 g, 76 %); m/z $[M+1]^+$ 367 (97 % purity), $\delta_H$ 2.6-3.8 (8H, m), 4.4 (2H, s), 7.2-7.5 (3H, m), 7.7-8.0 (3H, m), 8.2-8.3 (1H, m), 12.6 (1H, s).

*c. Synthesis of 4-[3-([1,4]diazepane-1-carbonyl)benzyl]-2H-phthalazin-1-one (3)*

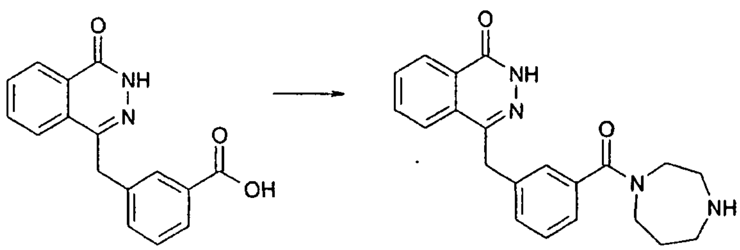

(A) (3)

The synthesis was carried out according to the method described in (a) above using 3-(4-oxo-3,4-dihydrophthalazin-1-ylmethyl)benzoic acid (A) and tert-butyl 1-homopiperazine carboxylate to yield 4-[3-([1,4]diazepane-1-carbonyl)benzyl]-2H-phthalazin-1-one (3) as a grey crystalline solid (5.3 g, 97 %); m/z $[M+1]^+$ 363 (97 % purity); $\delta_H$ 2.6-3.8 (10H, m), 4.4 (2H, s), 7.2-7.5 (4H, m), 7.7-8.0 (3H, m), 8.2-8.3 (1H, m), 12.6 (1H, s).

*d. Synthesis of 4-(3-((1,4)diazepane-1-carbonyl)-4-fluorobenzyl)-2H-phthalazin-1-one (4)*

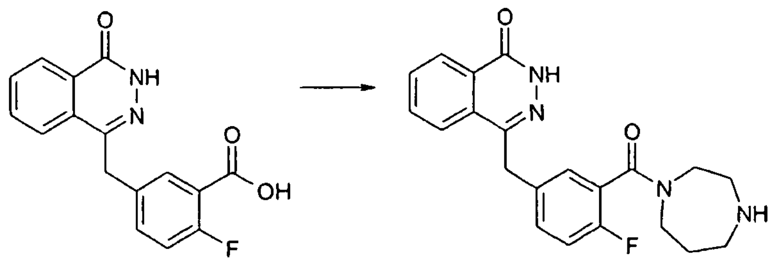

(B) (4)

The synthesis was carried out according to the method described in (a) above using 2-fluoro-5-(4-oxo-3,4-dihydrophthalazin-1-ylmethyl)benzoic acid (B) and tert-butyl 1-homopiperazinecarboxylate to yield 4-[3-([1,9]diazepane-1-carbonyl)benzyl]-2H-phthalazin-1-one (4) as a yellow crystalline solid (5.3 g, 68 %); m/z $[M+1]^+$ 381 (97 % purity); $\delta_H$ 2.6-3.8 (10H, m), 4.4 (2H, s), 7.2-7.5 (3H, m), 7.7-8.0 (3H, m), 8.2-8.3 (1H, m), 12.6 (1H, s).

**Example 2**

*a. 9-(3-(4-(6-Chlorobenzothiazol-2-yl)-1,9-diazepan-1-ylcarbonyl]benzyl)-1(2H)-phthalazinone*

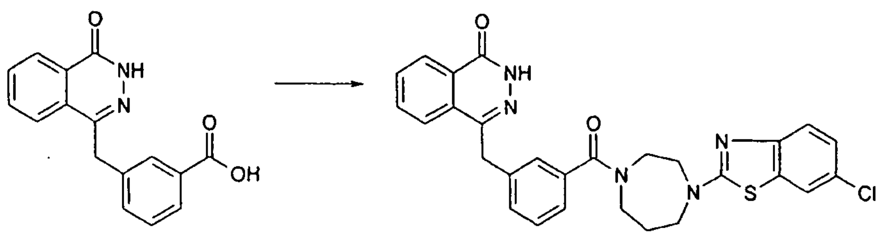

(A) (5)

2-(1H-Benzotriazol-1-yl)-1,1,3,3-tetramethyluronium tetrafluoroborate (150 mg, 0.47 mmol), diisopropylethylamine (102 mg, 0.8 mmol) and 6-chloro-2-(1,4-diazepan-1-yl)-1,3-benzothiazole (115 mg, 0.43 mmol) were added sequentially at ambient temperature to a stirred solution of 3-(4-oxo-3,4-dihydrophthalazin-1-ylmethyl)benzoic acid (A) (100 mg, 0.36 mmol) in dry dimethylacetamide (1 ml), the mixture was stirred at ambient temperature for 1 hour and allowed to stand at ambient temperature for 16 hours, then it was added dropwise to stirred cold water (10 ml). After 30 minutes, the resulting solid was collected by filtration, washed with water (2 x 1 ml) and hexane (1 ml), dried in vacuo and purified using preparative HPLC to give the desired compound (5)(166 mg) as a grey solid; HPLC purity 90%; HPLC Retention time 4.21 minutes; m/z $(M+H)^+$ 530.

b. The following compounds were synthesised in a manner analogous to that described in (a) above, but using appropriate alternative amine starting materials.

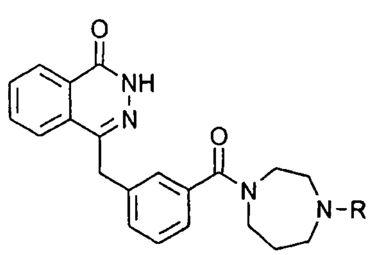

| Compound | R | LC RT (minutes) | M+1 | LC Purity (%) |
|---|---|---|---|---|
| 6 | 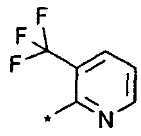 | 4.18 | 508 | 90 |
| 7 | 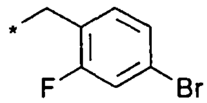 | 3.22 | 551 | 90 |
| 8 | 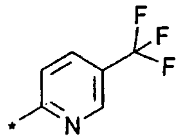 | 4.13 | 508 | 90 |
| 9 | 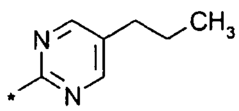 | 3.95 | 483 | 90 |
| 10 | 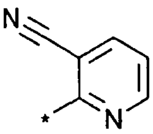 | 3.79 | 465 | 90 |
| 11 | 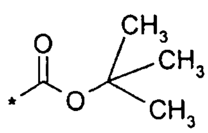 | 3.76 | 406 | 90 |
| 12 | 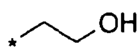 | 2.80 | 407 | 90 |

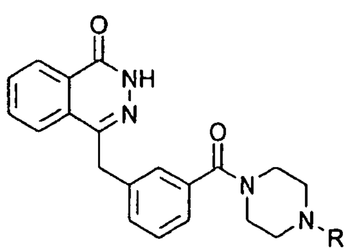

| Compound | R | LC RT (minutes) | M+1 | LC Purity (%) |
|---|---|---|---|---|
| 13 (Note 1) | 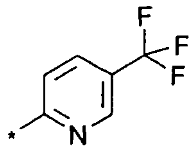 | 3.56 | 494 | 100 |
| 14 | 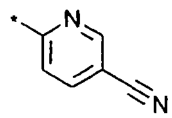 | 3.71 | 451 | 90 |
| 15 | 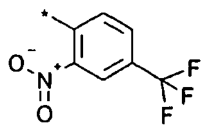 | 4.39 | 538 | 90 |
| 16 | 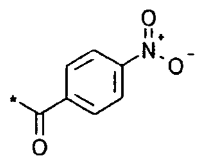 | 3.66 | 498 | 90 |
| 17 | 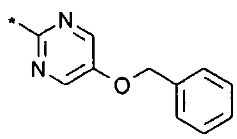 | 4.33 | 533 | 90 |
| Note 1: 13 did not require purification via preparative scale HPLC - the product from the reaction was essentially pure. | | | | |

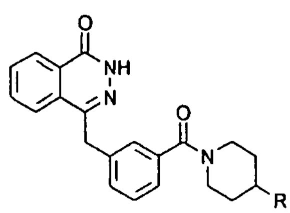

| Compound | R | LC RT (minutes) | M+1 | LC Purity (%) |
|---|---|---|---|---|
| 18 | 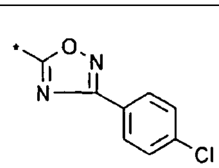 | 4.64 | 526 | 90 |
| 19 | 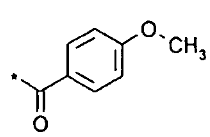 | 3.99 | 482 | 90 |

(continued)

| Compound | R | LC RT (minutes) | M+1 | LC Purity (%) |
| --- | --- | --- | --- | --- |
| 20 | 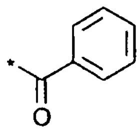 | 4.00 | 452 | 90 |
| 21 | 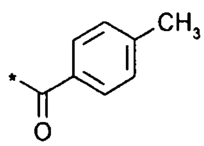 | 4.15 | 466 | 90 |

**Example 3**

*a. 4-{3-[4- (4-fluorophenyl) piperazin-1-ylcarbonyl]benzyl}-1(2H) - phthalazinone (22)*

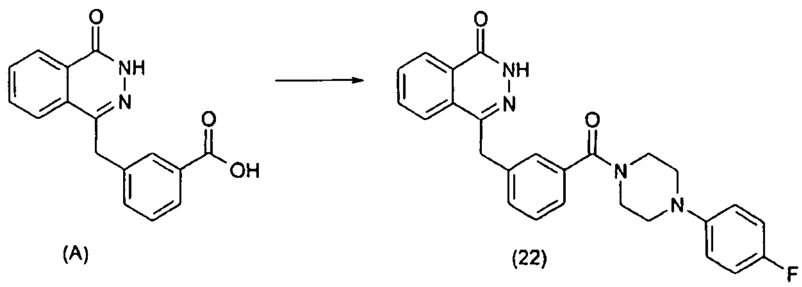

(A) (22)

2-(1H-Benzotriazol-1-yl)-1,1,3,3-tetramethyluronium tetrafluoroborate (150 mg, 0.47 mmol), diisopropylethylamine (102 mg, 0.8 mmol) and 1-(4-fluorophenyl)piperazine (65 mg, 0.47 mmol) were added sequentially at ambient temperature to a stirred solution of 3-(4-oxo-3,4-dihydrophthalazin-1-ylmethyl)benzoic acid (A)(100 mg, 0.36 mmol) in dry dimethylacetamide (1 ml), the mixture was stirred at ambient temperature for 4 hours and allowed to stand at ambient temperature for 16 hours, then it was added dropwise to stirred cold water (10 ml). After 30 minutes, the resulting solid was collected by filtration, washed with water (2 x 1 ml) and hexane (1 ml), dried in vacuo and purified using preparative HPLC to give 4-{3-[4-(4-fluorophenyl)piperazin-1-ylcarbonyl]benzyl}-1(2H)-phthalazinone (22)(76 mg) as a cream solid; m/z $(M+H)^+$ 443; HPLC Purity 90%; HPLC Retention time 4.00 minutes.

b. The following compounds were synthesised in a manner analogous to that described in (a) above, but using appropriate alternative amine starting materials.

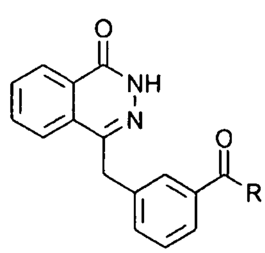

| Compound | R | LC RT (minutes) | M+1 | LC Purity (%) |
|---|---|---|---|---|
| 23 | 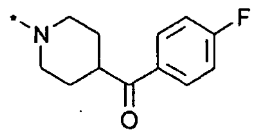 | 4.00 | 470 | 90 |
| 24 | 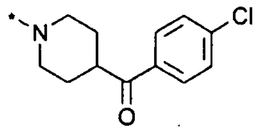 | 4.26 | 486 | 90 |
| 25 | 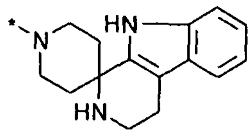 | 3.18 | 504 | 85 |
| 26 | 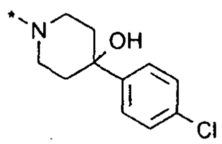 | 3.78 | 473 | 90 |
| 27 | 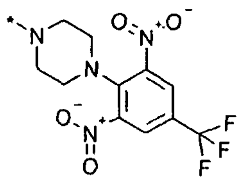 | 4.46 | 583 | 90 |
| 28 | 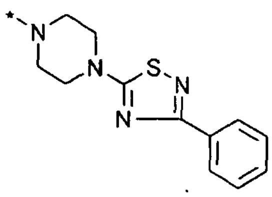 | 4.96 | 509 | 90 |
| 29 | 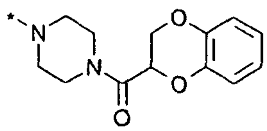 | 3.73 | 511 | 90 |
| 30 | 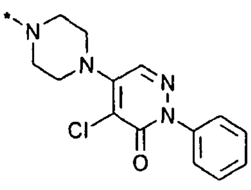 | 3.78 | 553 | 90 |
| 31 | 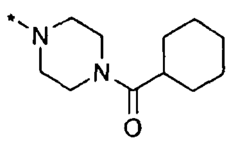 | 3.71 | 459 | 90 |

(continued)

| Compound | R | LC RT (minutes) | M+1 | LC Purity (%) |
|---|---|---|---|---|
| 32 | 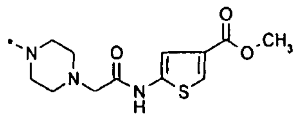 | 3.94 | 546 | 90 |
| 33 | 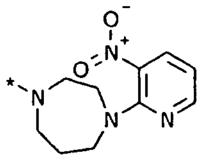 | 3.84 | 485 | 90 |
| 34 | 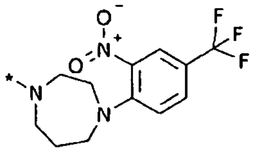 | 4.37 | 552 | 90 |
| 35 | 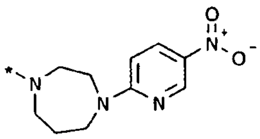 | 3.77 | 485 | 90 |
| 36 (Note 2) | 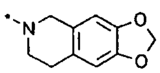 | 2.89 | 440 | 100 |
| Note 2: 36 did not require purification via preparative scale HPLC - the product from the reaction was essentially pure. | | | | |

**Example 4**

1-[3-(4-Oxo-3,4-dihydro-phthalazin-1-ylmethyl)-benzoyl]-piperidine-4-carboxylic acid (C) (0.24 mmol) was added to a solution of the appropriate amine (0.2 mmol) in dimethylacetamide (2 ml). 2-(1H-Benzotriazole-1-yl)-1,1,3,3-tetramethyluronium hexafluorophosphate (0.3 mmol) and Hunigs base (0.4 mmol) were then added and the reaction was stirred at room temperature for 16 hours. The reaction mixtures were then purified by preparative HPLC.

The compounds synthesised are set out below.

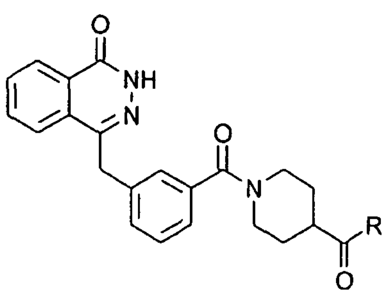

| Compound | R | LC RT (minutes) | M+1 | LC Purity (%) |
|---|---|---|---|---|
| 37 | 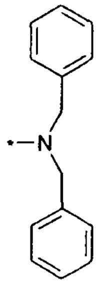 | 4.39 | 572 | 90 |
| 38 | 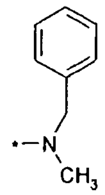 | 3.71 | 496 | 90 |
| 39 | 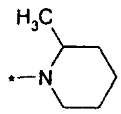 | 3.63 | 474 | 80 |
| 40 | 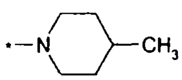 | 3.76 | 474 | 90 |
| 41 | 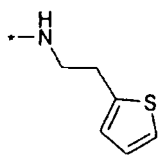 | 3.56 | 502 | 90 |
| 42 | 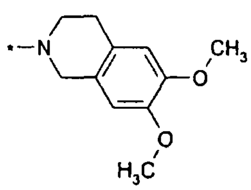 | 3.58 | 568 | 90 |
| 43 | 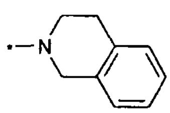 | 3.81 | 508 | 90 |
| 44 | 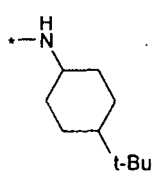 | 4.39 | 531 | 90 |
| 45 | 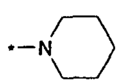 | 3.52 | 460 | 85 |

(continued)

| Compound | R | LC RT (minutes) | M+1 | LC Purity (%) |
|---|---|---|---|---|
| 46 | 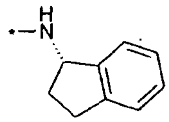 | 3.77 | 508 | 90 |
| 47 | 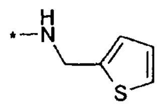 | 3.59 | 488 | 90 |
| 48 | 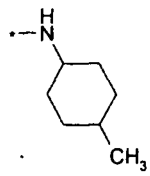 | 3.83 | 488 | 90 |
| 49 | 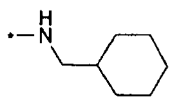 | 3.85 | 488 | 90 |
| 50 | 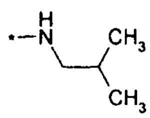 | 3.47 | 448 | 90 |
| 51 | 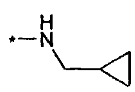 | 3.36 | 446 | 90 |
| 52 | 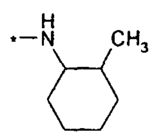 | 3.77 | 488 | 90 |
| 53 | 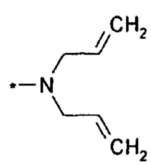 | 3.74 | 472 | 90 |
| 54 | 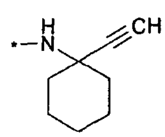 | 3.82 | 498 | 90 |
| 55 | 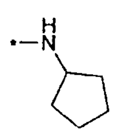 | 3.52 | 460 | 90 |

(continued)

| Compound | R | LC RT (minutes) | M+1 | LC Purity (%) |
|---|---|---|---|---|
| 56 | 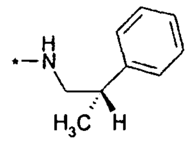 | 3.86 | 510 | 90 |
| 57 | 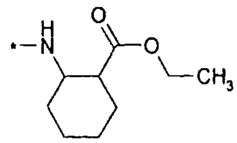 | 3.75 | 546 | 90 |
| 58 | 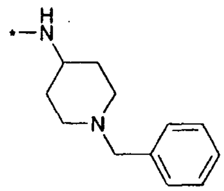 | 3.01 | 565 | 90 |
| 59 | 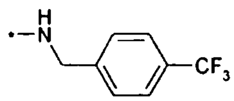 | 3.93 | 549 | 90 |
| 60 | 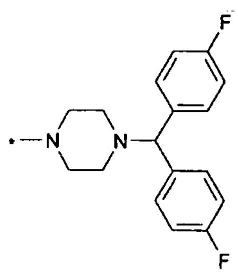 | 4.17 | 663 | 90 |
| 61 | 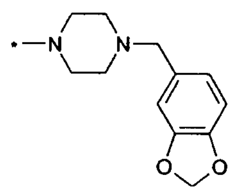 | 3.08 | 595 | 90 |
| 62 | 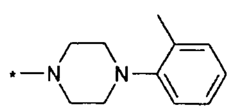 | 4.16 | 551 | 90 |
| 63 | 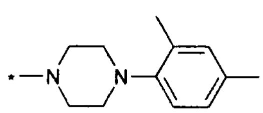 | 4.3 | 565 | 90 |
| 64 | 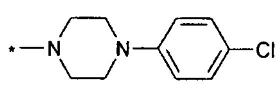 | 4.1 | 571 | 90 |

(continued)

| Compound | R | LC RT (minutes) | M+1 | LC Purity (%) |
|---|---|---|---|---|
| 65 | 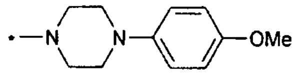 | 3.64 | 567 | 90 |
| 66 | 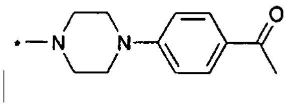 | 3.62 | 579 | 90 |
| 67 | 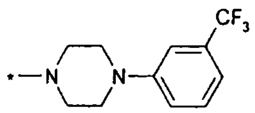 | 4.27 | 605 | 90 |
| 68 | 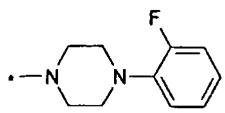 | 3.89 | 555 | 90 |
| 69 | 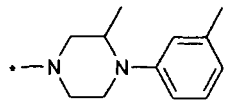 | 3.84 | 565 | 90 |
| 70 | 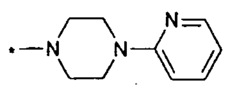 | 2.92 | 565 | 90 |
| 71 | 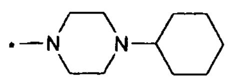 | 3.02 | 543 | 90 |

**Example 5**

1-[2-Fluoro-5-(4-Oxo-3,4-dihydrophthalazin-1-ylmethyl)benzoyl]-piperidine-4-carboxylic acid (D) (0.24 mmol) was added to a solution of the appropriate amine (0.2 mmol) in dimethylacetamide (2 ml). 2-(1H-Benzotriazole-1-yl)-1,1,3,3-tetramethyluronium hexafluorophosphate (0.3 mmol) and Hunigs base (0.4 mmol) were then added and the reaction was stirred at room temperature for 16 hours. The reaction mixtures were then purified by preparative HPLC.

The compounds synthesised are set out below.

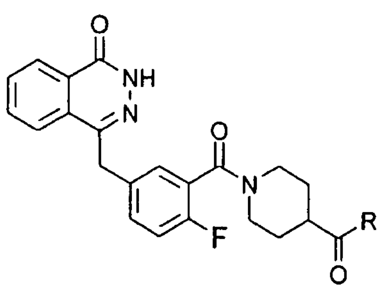

| Compound | R | LC RT (minutes ) | M+1 | LC Purity (%) |
|---|---|---|---|---|
| 72 | 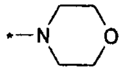 | 3.21 | 480 | 90 |

**Example 6**

The appropriate sulphonyl chloride (0.24 mmol) was added to a solution of 4-[3-(piperazine-1-carbonyl)benzyl]-2H-phthalazin-1-one (1) (0.2 mmol) in dichloromethane (2 ml). Hunigs base (0.4 mmol) was then added and the reaction was stirred at room temperature for 16 hours. The reaction mixtures were then purified by preparative HPLC.

The compounds synthesised are set out below.

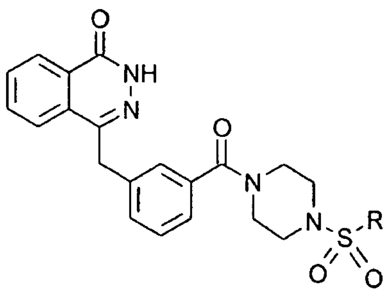

| Compound | R | LC RT (minutes) | M+1 | LC Purity (%) |
|---|---|---|---|---|
| 73 | 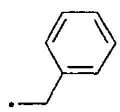 | 3.85 | 504 | 90 |
| 74 | 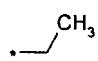 | 3.44 | 442 | 90 |
| 75 | 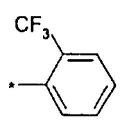 | 4.09 | 558 | 90 |
| 76 | 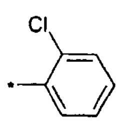 | 3.93 | 525 | 90 |
| 77 | 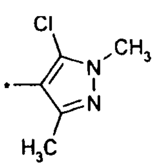 | 3.73 | 543 | 90 |

(continued)

| Compound | R | LC RT (minutes) | M+1 | LC Purity (%) |
|---|---|---|---|---|
| 78 | 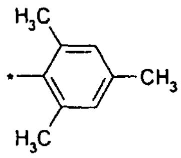 | 4.38 | 532 | 90 |
| 79 | 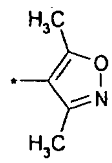 | 3.76 | 509 | 90 |
| 80 | 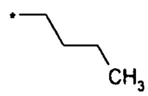 | 3.82 | 470 | 90 |

**Example 7**

The appropriate sulphonyl chloride (0.24 mmol) was added to a solution of 9-[3-([1,4]diazepane-1-carbonyl) benzyl]-2H-phthalazin-1-one (3) (0.2 mmol) in dichloromethane (2 ml). Hunigs base (0.4 mmol) was then added and the reaction was stirred at room temperature for 16 hours. The reaction mixtures were then purified by preparative HPLC.
The compounds synthesised are set out below.

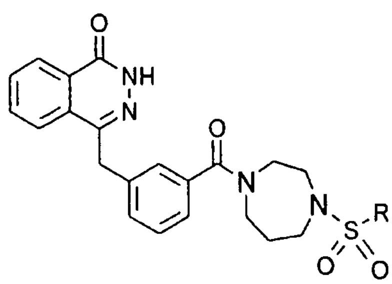

| Compound | R | LC RT (minutes) | M+1 | LC Purity (%) |
|---|---|---|---|---|
| 81 | 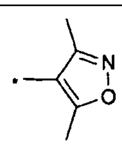 | 3.71 | 523 | 90 |
| 82 | 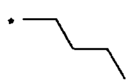 | 3.68 | 484 | 90 |

**Example 8**

The appropriate acid chloride (0.24 mmol) was added to a solution of 4-[3-(piperazine-1-carbonyl)benzyl]-2H-phthalazin-1-one (1) (0.2 mmol) in dichloromethane (2 ml). Hunigs base (0.4 mmol) was then added and the reaction was stirred at room temperature for 16 hours. The reaction mixtures were then purified by preparative HPLC.
The compounds synthesised are set out below.

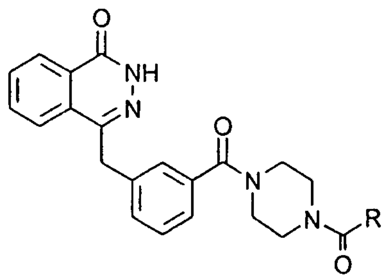

| Compound | R | LC RT (minute s) | M+1 | LC Purity (%) |
|---|---|---|---|---|
| 83 | 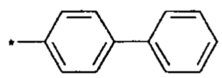 | 4.04 | 530 | 85 |
| 84 | 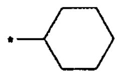 | 3.71 | 460 | 90 |
| 85 | 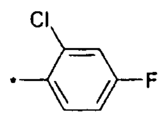 | 3.69 | 506 | 90 |
| 86 | 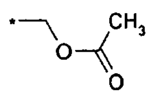 | 3.19 | 450 | 85 |
| 87 | 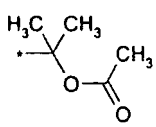 | 3.44 | 478 | 90 |
| 88 | 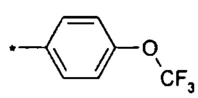 | 3.94 | 538 | 90 |
| 89 | 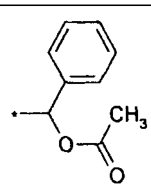 | 3.68 | 526 | 90 |
| 90 | 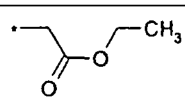 | 3.6 | 464 | 90 |
| 91 | 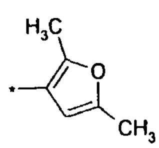 | 3.63 | 472 | 90 |

(continued)

| Compound | R | LC RT (minute s) | M+1 | LC Purity (%) |
|---|---|---|---|---|
| 92 | 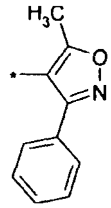 | 3.73 | 535 | 80 |
| 93 | 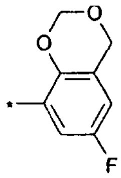 | 3.49 | 530 | 90 |
| 94 | 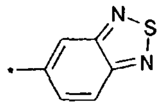 | 3.5 | 512 | 85 |
| 95 | 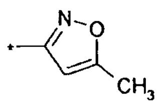 | 3.52 | 459 | 90 |
| 96 | 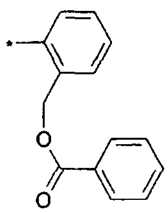 | 4.01 | 588 | 90 |
| 97 | 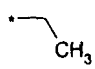 | 4.05 | 406 | 90 |
| 98 | 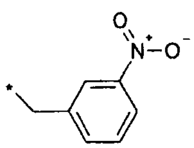 | 3.84 | 513 | 90 |
| 99 | 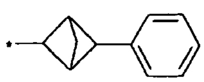 | 4.07 | 520 | 90 |
| 100 | 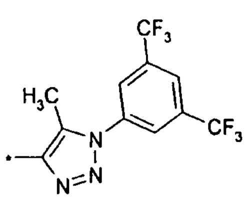 | 4.41 | 671 | 90 |

(continued)

| Compound | R | LC RT (minute s) | M+1 | LC Purity (%) |
|---|---|---|---|---|
| 101 | 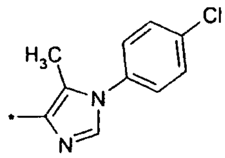 | 3.62 | 582 | 90 |
| 102 | 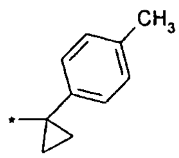 | 3.82 | 508 | 90 |
| 103 | 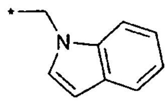 | 3.81 | 507 | 90 |
| 104 | 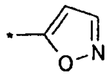 | 3.33 | 445 | 90 |
| 105 | 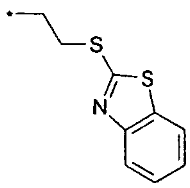 | 4.08 | 571 | 90 |
| 106 | 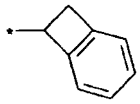 | 3.67 | 480 | 90 |
| 107 | 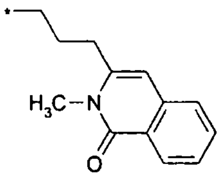 | 3.54 | 577 | 90 |
| 108 | 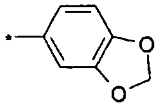 | 3.49 | 498 | 90 |
| 109 | 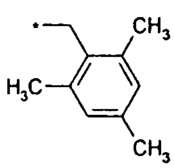 | 4.04 | 510 | 90 |
| 110 | 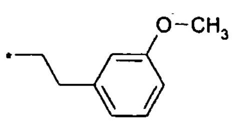 | 3.75 | 512 | 90 |

(continued)

| Compound | R | LC RT (minute s) | M+1 | LC Purity (%) |
|---|---|---|---|---|
| 111 | 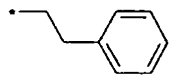 | 3.67 | 482 | 90 |
| 112 | 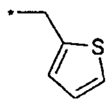 | 3.54 | 474 | 90 |
| 113 | 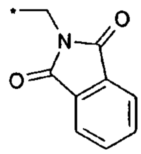 | 3.52 | 537 | 90 |
| 114 | 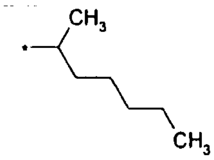 | 4.13 | 475 | 90 |
| 115 | 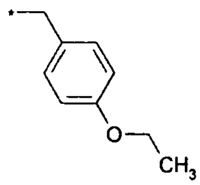 | 3.8 | 512 | 85 |
| 116 | 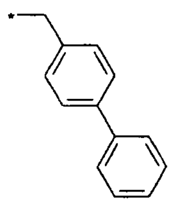 | 4.09 | 544 | 90 |
| 117 | 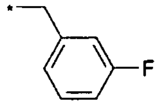 | 3.63 | 486 | 90 |
| 118 | 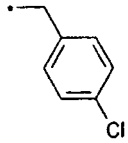 | 3.91 | 502 | 90 |
| 119 | 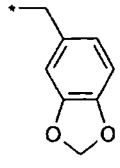 | 3.61 | 511 | 90 |
| 120 | 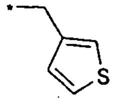 | 3.57 | 474 | 90 |

(continued)

| Compound | R | LC RT (minute s) | M+1 | LC Purity (%) |
|---|---|---|---|---|
| 121 | 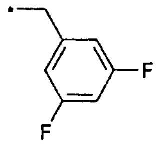 | 3.67 | 504 | 90 |
| 122 | 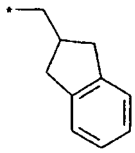 | 4.02 | 508 | 90 |
| 123 | 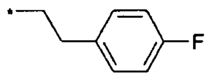 | 3.81 | 500 | 90 |
| 124 | 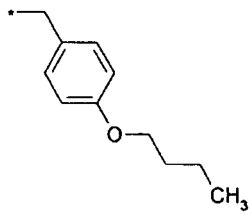 | 4.11 | 540 | 90 |
| 125 | 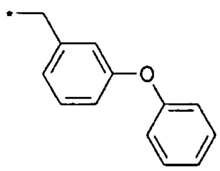 | 4.19 | 560 | 90 |
| 126 | 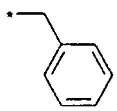 | 3.61 | 468 | 90 |
| 127 | 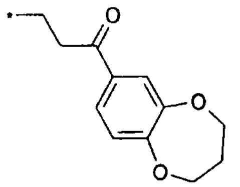 | 3.69 | 582 | 90 |
| 128 | 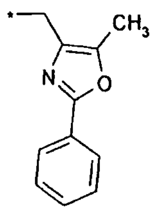 | 3.85 | 549 | 85 |
| 129 | 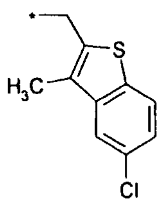 | 4.37 | 573 | 90 |

(continued)

| Compound | R | LC RT (minute s) | M+1 | LC Purity (%) |
|---|---|---|---|---|
| 130 | 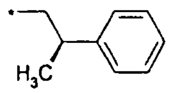 | 3.84 | 496 | 90 |
| 131 | 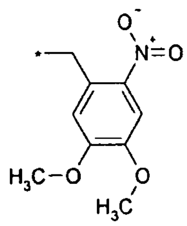 | 3.62 | 573 | 90 |
| 132 | 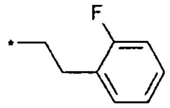 | 3.72 | 500 | 90 |
| 133 | 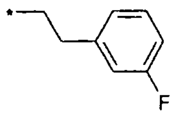 | 3.8 | 500 | 85 |
| 134 | 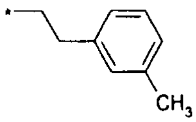 | 3.9 | 496 | 90 |
| 135 | 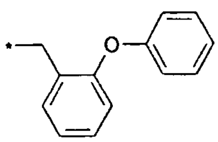 | 4.03 | 560 | 90 |
| 136 | 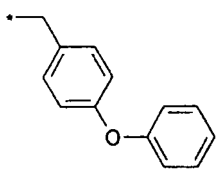 | 4.16 | 560 | 90 |
| 137 | 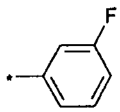 | 4.71 | 472 | 80 |
| 138 | 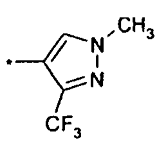 | 3.47 | 526 | 90 |

(continued)

| Compound | R | LC RT (minute s) | M+1 | LC Purity (%) |
|---|---|---|---|---|
| 139 | 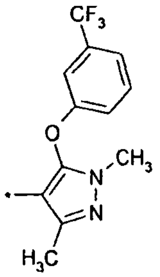 | 3.78 | 632 | 90 |
| 140 | 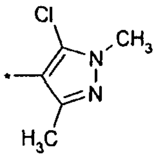 | 3.27 | 506 | 90 |
| 141 | 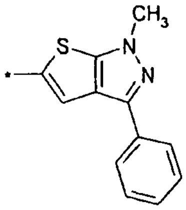 | 3.92 | 590 | 90 |
| 142 | 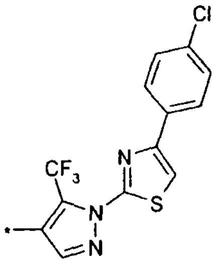 | 4.76 | 706 | 90 |
| 143 | 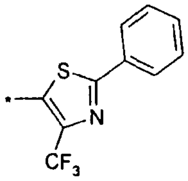 | 4.27 | 605 | 90 |
| 144 | 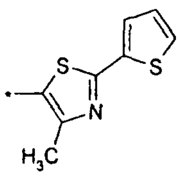 | 3.71 | 557 | 90 |
| 145 | 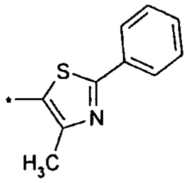 | 3.98 | 551 | 90 |

(continued)

| Compound | R | LC RT (minute s) | M+1 | LC Purity (%) |
|---|---|---|---|---|
| 146 | 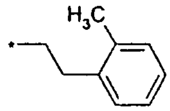 | 3.9 | 496 | 90 |
| 147 | 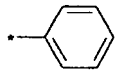 | 3.57 | 454 | 90 |
| 148 | 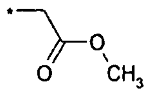 | 3.21 | 450 | 90 |
| 149 | 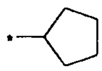 | 3.61 | 446 | 90 |
| 150 | 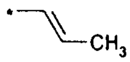 | 3.39 | 418 | 85 |
| 151 | 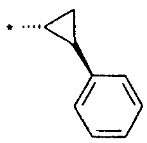 | 3.81 | 494 | 90 |
| 152 | 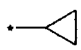 | 3.31 | 418 | 90 |
| 153 | 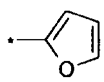 | 3.38 | 444 | 90 |
| 154 | 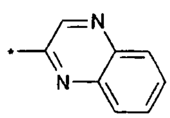 | 3.56 | 506 | 85 |
| 155 | 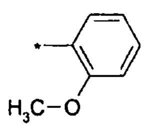 | 3.48 | 484 | 90 |
| 156 | 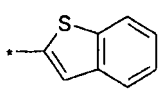 | 3.84 | 510 | 90 |
| 157 | 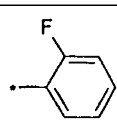 | 3.56 | 472 | 90 |
| 158 | 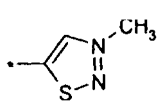 | 3.34 | 476 | 90 |

(continued)

| Compound | R | LC RT (minute s) | M+1 | LC Purity (%) |
|---|---|---|---|---|
| 159 | 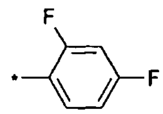 | 3.56 | 490 | 90 |
| 160 | 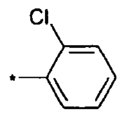 | 3.53 | 488 | 90 |
| 161 | 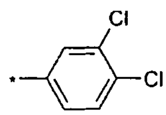 | 3.99 | 523 | 90 |
| 162 | 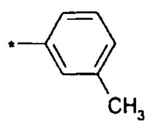 | 3.7 | 468 | 90 |
| 163 | *-$CH_3$ | 3.11 | 392 | 90 |
| 164 | 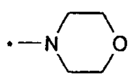 | 3.16 | 463 | 90 |
| 165 | 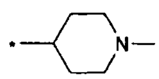 | 2.78 | 475 | 90 |

**Example 9**

The appropriate acid chloride (0.24 mmol) was added to a solution of 9-[4-Fluoro-3-(piperazine-1-carbonyl) benzyl]-2H-phthalazin-1-one (2) (0.2 mmol) in dichloromethane (2 ml). Hunigs base (0.4 mmol) was then added and the reaction was stirred at room temperature for 16 hours. The reaction mixtures were then purified by preparative HPLC.

The compounds synthesised are set out below.

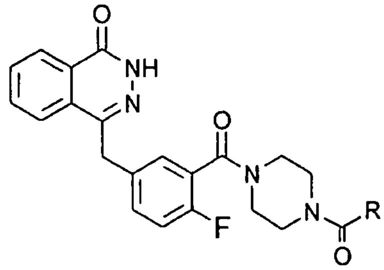

| Compound | R | LC RT (minutes) | M+1 | LC Purity (%) |
|---|---|---|---|---|
| 166 | 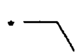 | 4.04 | 424 | 90 |

(continued)

| Compound | R | LC RT (minutes) | M+1 | LC Purity (%) |
|---|---|---|---|---|
| 167 | 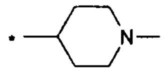 | 2.84 | 493 | 90 |
| 168 | 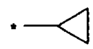 | 4.14 | 436 | 90 |
| 169 | 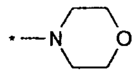 | 3.19 | 481 | 90 |
| 170 | 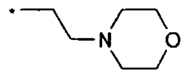 | 2.83 | 509 | 90 |
| 171 | 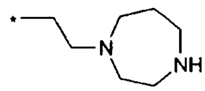 | 2.63 | 522 | 90 |
| 172 | 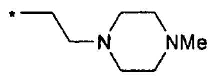 | 2.71 | 522 | 90 |
| 173 | 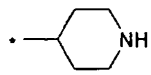 | 2.8 | 479 | 90 |

**Example 10**

The appropriate acid chloride (0.24 mmol) was added to a solution of 9-[3-([1,4]diazepane-1-carbonyl)benzyl]-2H-phthalazin-1-one (3) (0.2 mmol) in dichloromethane (2 ml). Hunigs base (0.4 mmol) was then added and the reaction was stirred at room temperature for 16 hours. The reaction mixtures were then purified by preparative HPLC.

The compounds synthesised are set out below.

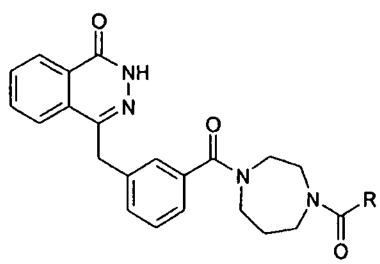

| Compound | R | LC RT (minutes) | M+1 | LC Purity (%) |
|---|---|---|---|---|
| 174 | 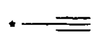 | 3.16 | 416 | 90 |
| 175 | 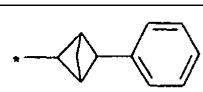 | 3.84 | 534 | 90 |
| 176 | 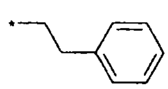 | 3.63 | 496 | 90 |

(continued)

| Compound | R | LC RT (minutes) | M+1 | LC Purity (%) |
|---|---|---|---|---|
| 177 | 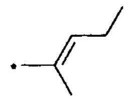 | 3.52 | 460 | 90 |
| 178 | 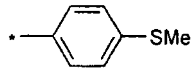 | 3.62 | 514 | 90 |
| 179 | 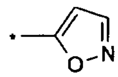 | 3.23 | 459 | 90 |
| 180 | 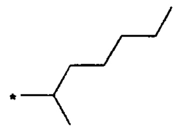 | 3.95 | 490 | 90 |
| 181 | 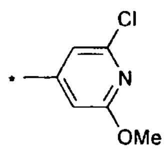 | 3.63 | 533 | 90 |
| 182 | 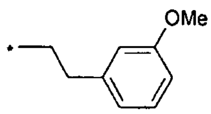 | 3.61 | 526 | 90 |

**Example 11**

The appropriate acid chloride (0.24 mmol) was added to a solution of 4-[3-([1,4]diazepane-1-carbonyl)-4-fluorobenzyl]-2H-phthalazin-1-one (4) (0.2 mmol) in dichloromethane (2 ml). Hunigs base (0.4 mmol) was then added and the reaction was stirred at room temperature for 16 hours. The reaction mixtures were then purified by preparative HPLC.

The compounds synthesised are set out below.

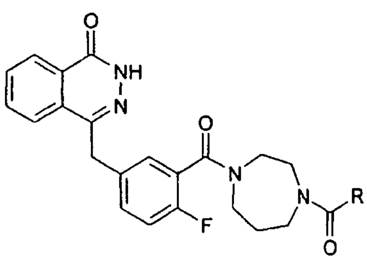

| Compound | R | LC RT (minutes) | M+1 | LC Purity (%) |
|---|---|---|---|---|
| 183 | 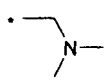 | 2.81 | 467 | 90 |
| 184 | 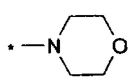 | 3.19 | 495 | 90 |

**Example 12**

The appropriate isocyanate (0.24 mmol) was added to a solution of 4-[3-(piperazine-1-carbonyl)benzyl]-2H-phthalazin-1-one (1) (0.2 mmol) in dichloromethane (2 ml). The reaction was stirred at room temperature for 16 hours. The reaction mixtures were then purified by preparative HPLC.

The compounds synthesised are set out below.

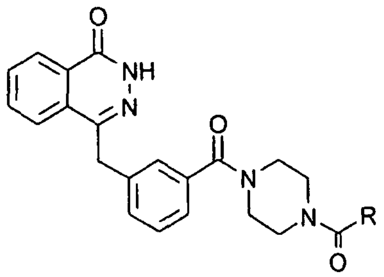

| Compound | R | LC RT (minute s) | M+1 | LC Purity (%) |
|---|---|---|---|---|
| 185 | 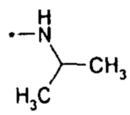 | 3.34 | 435 | 85 |
| 186 | 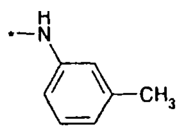 | 3.76 | 483 | 90 |
| 187 | 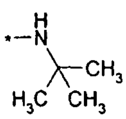 | 3.49 | 449 | 90 |
| 188 | 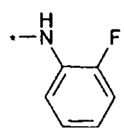 | 3.57 | 487 | 90 |
| 189 | 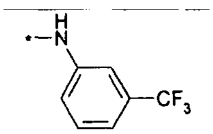 | 4.03 | 537 | 90 |
| 190 | 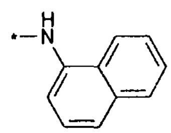 | 3.77 | 519 | 85 |
| 191 | 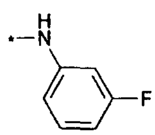 | 3.72 | 487 | 90 |

(continued)

| Compound | R | LC RT (minute s) | M+1 | LC Purity (%) |
|---|---|---|---|---|
| 192 | 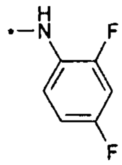 | 3.57 | 505 | 90 |
| 193 | 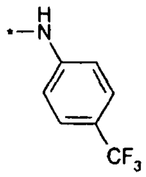 | 4.08 | 537 | 80 |
| 194 | 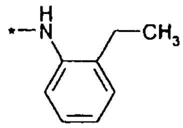 | 3.68 | 497 | 85 |
| 195 | 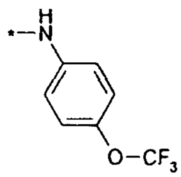 | 4.03 | 553 | 90 |
| 196 | 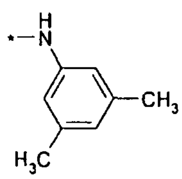 | 3.93 | 497 | 90 |
| 197 | 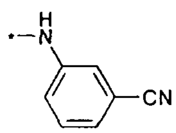 | 3.62 | 494 | 90 |
| 198 | 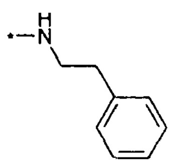 | 3.68 | 497 | 90 |
| 199 | 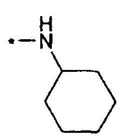 | 3.73 | 475 | 90 |

(continued)

| Compound | R | LC RT (minute s) | M+1 | LC Purity (%) |
|---|---|---|---|---|
| 200 | 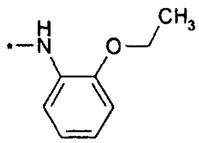 | 3.9 | 513 | 90 |
| 201 | 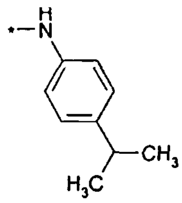 | 4.11 | 511 | 90 |
| 202 | 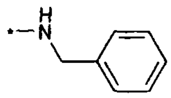 | 3.58 | 483 | 90 |
| 203 | 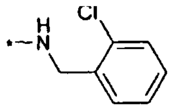 | 3.71 | 517 | 90 |
| 204 | 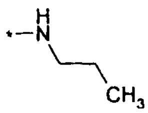 | 3.34 | 435 | 85 |
| 205 | 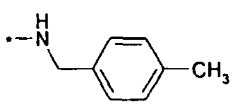 | 3.71 | 497 | 90 |
| 206 | 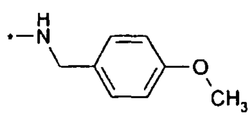 | 3.56 | 513 | 90 |
| 207 | 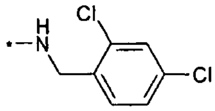 | 4.04 | 552 | 90 |
| 208 | 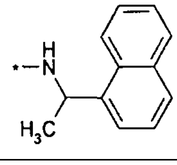 | 4 | 547 | 90 |
| 209 | 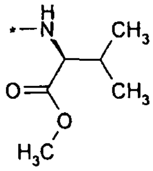 | 3.54 | 507 | 90 |

(continued)

| Compound | R | LC RT (minute s) | M+1 | LC Purity (%) |
|---|---|---|---|---|
| 210 | 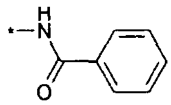 | 3.42 | 497 | 90 |
| 211 | 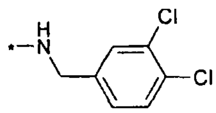 | 3.95 | 552 | 90 |
| 212 | 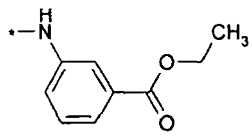 | 3.79 | 541 | 85 |
| 213 | 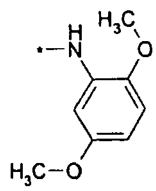 | 3.66 | 529 | 90 |
| 214 | 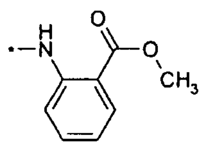 | 3.92 | 527 | 85 |
| 215 | 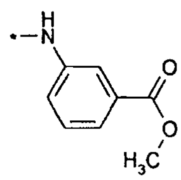 | 3.62 | 527 | 90 |
| 216 | 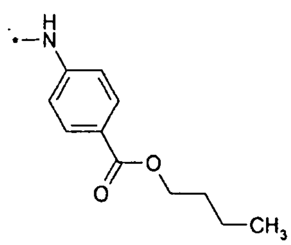 | 4.28 | 569 | 90 |
| 217 | 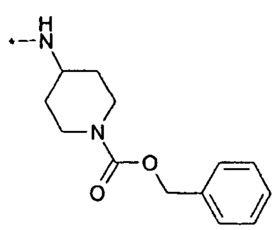 | 3.81 | 610 | 90 |

**Example 13**

The appropriate isothiocyanate (0.24 mmol) was added to a solution of 4-[3-(piperazine-1-carbonyl)benzyl]-2H-phthalazin-1-one (1) (0.2 mmol) in dichloromethane (2 ml). The reaction was stirred at room temperature for 16 hours. The reaction mixtures were then purified by preparative HPLC.

The compounds synthesised are set out below.

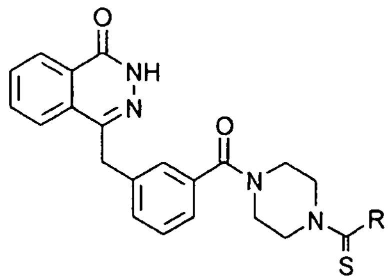

| Compound | R | LC RT (minute s) | M+1 | LC Purity (%) |
|---|---|---|---|---|
| 218 | 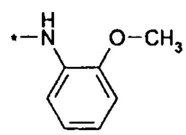 | 3.68 | 515 | 90 |
| 219 | 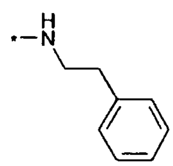 | 4.05 | 513 | 90 |
| 220 | 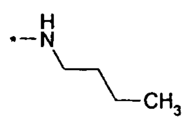 | 3.94 | 465 | 90 |
| 221 | 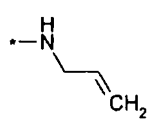 | 3.55 | 449 | 90 |
| 222 | 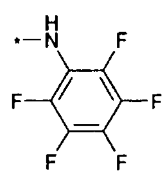 | 4.21 | 575 | 90 |
| 223 | 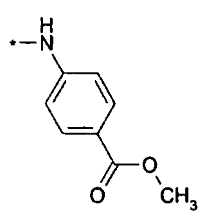 | 3.79 | 543 | 90 |

(continued)

| Compound | R | LC RT (minute s) | M+1 | LC Purity (%) |
|---|---|---|---|---|
| 224 | 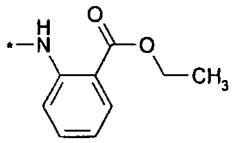 | 4.28 | 557 | 85 |
| 225 | 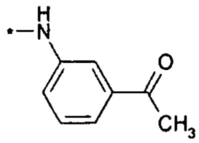 | 3.63 | 527 | 90 |
| 226 | 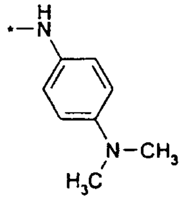 | 3.18 | 528 | 90 |
| 227 | 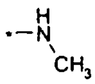 | 3.32 | 423 | 90 |
| 228 | 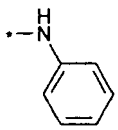 | 3.69 | 485 | 80 |
| 229 | 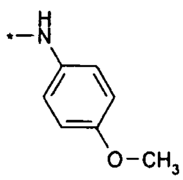 | 3.68 | 515 | 90 |
| 230 | 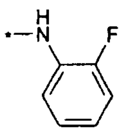 | 3.72 | 503 | 90 |

**Example 14**

3-(4-Oxo-3,4-dihydrophthalazin-1-ylmethyl)benzoic acid (A) (0.24 mmol) was added to a solution of the appropriate amine (0.2 mmol) in dimethylacetamide (2 ml). 2-(1H-Benzotriazol-1-yl)-1,1,3,3-tetramethyluronium hexafluorophosphate (0.3 mmol) and Hunigs base (0.4 mmol) were then added and the reaction was stirred at room temperature for 16 hours. The reaction mixtures were then purified by preparatory HPLC.

The compounds synthesised are set out below.

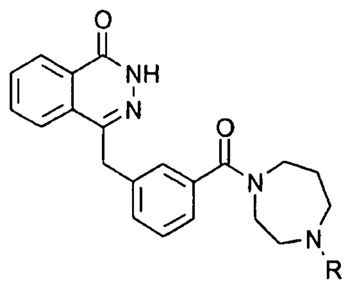

| Compound | R | LC RT (minute s) | M+1 | LC Purity (%) |
|---|---|---|---|---|
| 231 | $*-CH_3$ | 2.72 | 378 | 90 |

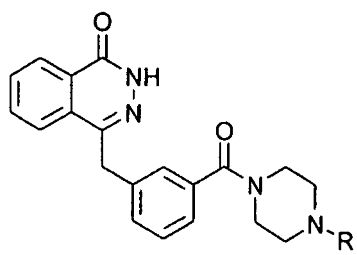

| Compound | R | LC RT (minutes) | M+1 | LC Purity (%) |
|---|---|---|---|---|
| 232 | 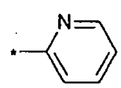 | 2.96 | 427 | 90 |
| 233 | 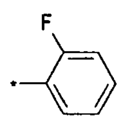 | 4.08 | 444 | 90 |
| 234 | 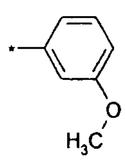 | 3.9 | 456 | 95 |
| 235 | 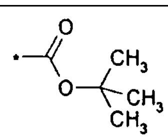 | 3.83 | 450 | 95 |
| 236 | 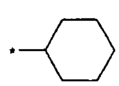 | 2.98 | 432 | 90 |
| 237 | 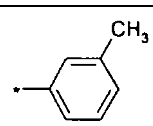 | 4.17 | 440 | 90 |

(continued)

| Compound | R | LC RT (minutes) | M+1 | LC Purity (%) |
|---|---|---|---|---|
| 238 | 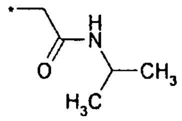 | 2.9 | 449 | 90 |
| 239 | 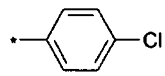 | 4.31 | 460 | 90 |
| 240 | 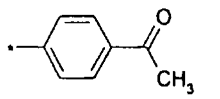 | 3.63 | 468 | 90 |
| 241 | 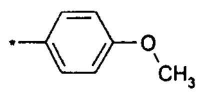 | 3.78 | 456 | 90 |
| 242 | 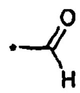 | 3.08 | 378 | 90 |

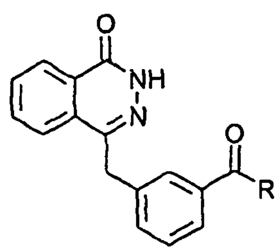

| Compound | R | LC RT (minutes) | M+1 | LC Purity (%) |
|---|---|---|---|---|
| 243 | 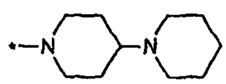 | 2.88 | 432 | 90 |
| 244 | 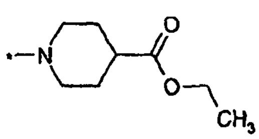 | 3.61 | 421 | 95 |

**Example 15**

2-Fluoro-5-(4-oxo-3,4-dihydrophthalazin-1-ylmethyl)benzoic acid (B) (0.24 mmol) was added to a solution of the appropriate amine (0.2 mmol) in dimethylacetamide (2 ml). 2-(1H-Benzotriazol-1-yl)-1,1,3,3-tetramethyluronium hexafluorophosphate (0.3 mmol) and Hunigs base (0.4 mmol) were then added and the reaction was stirred at room temperature for 16 hours. The reaction mixtures were then purified by preparative HPLC.

The compounds synthesised are set out below.

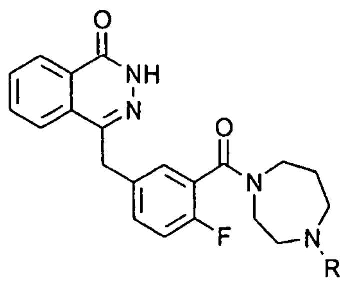

| Compound | R | LC RT (minute s) | M+1 | LC Purity (%) |
|---|---|---|---|---|
| 246 | $*-CH_3$ | 2.8 | 396 | 90 |
| 247 | 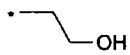 | 2.79 | 426 | 90 |

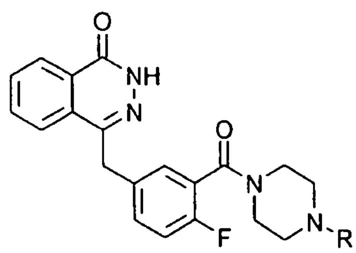

| Compound | R | LC RT (minutes) | M+1 | LC Purity (%) |
|---|---|---|---|---|
| 248 | 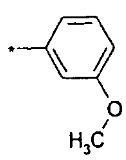 | 3.9 | 456 | 95 |
| 249 | 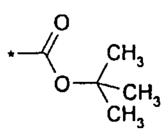 | 3.97 | 467 | 90 |
| 250 | 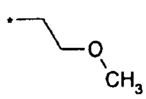 | 2.84 | 426 | 90 |
| 251 | $*-CH_3$ | 3.46 | 368 | 90 |
| 252 | 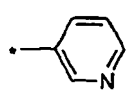 | 2.91 | 445 | 90 |
| 253 | 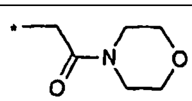 | 2.77 | 495 | 90 |

(continued)

| Compound | R | LC RT (minutes) | M+1 | LC Purity (%) |
|---|---|---|---|---|
| 254 | 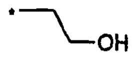 | 2.69 | 412 | 90 |
| 255 | 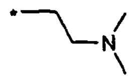 | 2.76 | 439 | 90 |

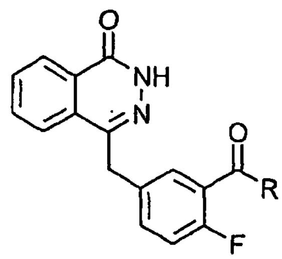

| Compound | R | LC RT (minutes) | M+1 | LC Purity (%) |
|---|---|---|---|---|
| 256 | 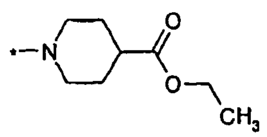 | 3.81 | 439 | 90 |

**Example 16**

An appriopriate aldehyde (0.2 mmol) and 4-[3-(piperazine-1-carbonyl)benzyl]-2H-phthalazin-1-one (1)(0.24 mmol) were dissolved in dichloromethane (2 ml). Sodium triacetoxyborohydride (0.28 mmol) and glacial acetic acid (6.0 mmol) were then added and stirred at room temperature for 16 hours. The reaction mixtures were then purified by preparative HPLC.

The compounds synthesised are set out below.

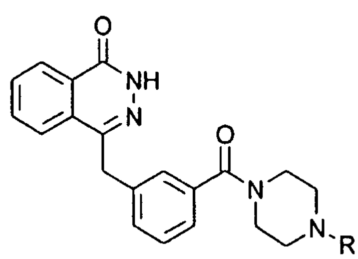

| Compound | R | LC RT (minutes) | M+1 | LC Purity (%) |
|---|---|---|---|---|
| 258 | 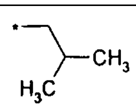 | 2.89 | 406 | 90 |

(continued)

| Compound | R | LC RT (minutes) | M+1 | LC Purity (%) |
|---|---|---|---|---|
| 259 | 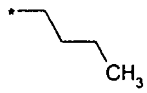 | 2.91 | 406 | 90 |
| 260 | 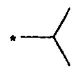 | 2.75 | 392 | 90 |
| 261 | 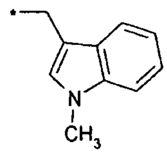 | 3.16 | 493 | 90 |
| 262 | 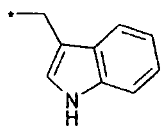 | 3.09 | 479 | 90 |

**Example 17**

An appriopriate aldehyde (0.2 mmol) and 4-[4-Fluoro-3-(piperazine-1-carbonyl)benzyl]-2H-phthalazin-1-one (2)(0.24 mmol) were dissolved in dichloromethane (2 ml). Sodium triacetoxyborohydride (0.28 mmol) and glacial acetic acid (6.0 mmol) were then added and stirred at room temperature for 16 hours. The reaction mixtures were then purified by preparative HPLC.

The compounds synthesised are set out below.

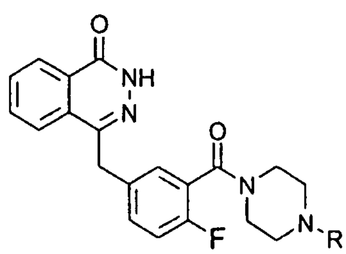

| Compound | R | LC RT (minutes) | M+1 | LC Purity (%) |
|---|---|---|---|---|
| 263 | 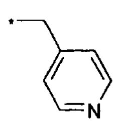 | 2.83 | 459 | 90 |
| 264 | 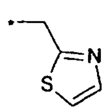 | 3.04 | 465 | 90 |

**Example 18**

An appriopriate aldehyde (0.2 mmol) and 4-[3-([1,4]diazepane-1-carbonyl)benzyl]-2H-phthalazin-1-one (3) (0.24 mmol) were dissolved in dichloromethane (2 ml). Sodium triacetoxyborohydride (0.28 mmol) and glacial acetic acid (6.0 mmol) were then added and stirred at room temperature for 16 hours. The reaction mixtures were then purified

by preparative HPLC.

The compounds synthesised are set out below.

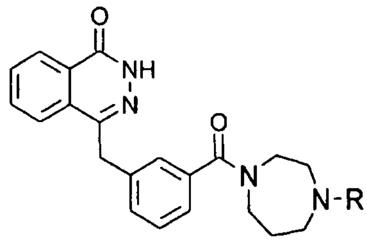

| Compound | R | LC RT (minutes) | M+1 | LC Purity (%) |
|---|---|---|---|---|
| 265 | 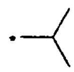 | 2.76 | 406 | 90 |

**Example 19**

An appriopriate aldehyde (0.2 mmol) and 4-[3-([1,4]diazepane-1-carbonyl)-4-fluorobenzyl]-2H-phthalazin-1-one (4)(0.24 mmol) were dissolved in dichloromethane (2 ml). Sodium triacetoxyborohydride (0.28 mmol) and glacial acetic acid (6.0 mmol) were then added and stirred at room temperature for 16 hours. The reaction mixtures were then purified by preparative HPLC.

The compounds synthesised are set out below.

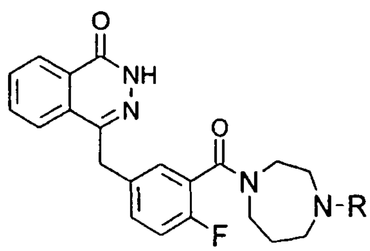

| Compound | R | LC RT (minutes) | M+1 | LC Purity (%) |
|---|---|---|---|---|
| 266 | 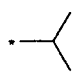 | 2.8 | 424 | 90 |

**Example 20**

In order to assess the inhibitory action of the compounds, the following assay was used to determine $IC_{50}$ values.

Mammalian PARP, isolated from Hela cell nuclear extract, was incubated with Z-buffer (25mM Hepes (Sigma); 12.5 mM $MgCl_2$ (Sigma); 50mM KCl (Sigma); 1 mM DTT (Sigma); 10% Glycerol (Sigma) 0.001% NP-40 (Sigma); pH 7.4) in 96 well FlashPlates (TRADE MARK) (NEN, UK) and varying concentrations of said inhibitors added. All compounds were diluted in DMSO and gave final assay concentrations of between 10 and 0.01 μM, with the DMSO being at a final concentration of 1% per well. The total assay volume per well was 40 μl.

After 10 minutes incubation at 30°C the reactions were initiated by the addition of a 10 μl reaction mixture, containing NAD (5μM), $^3$H-NAD and 30mer double stranded DNA-oligos. Designated positive and negative reaction wells were done in combination with compound wells (unknowns) in order to calculate % enzyme activities. The plates were then shaken for 2 minutes and incubated at 30°C for 45 minutes.

Following the incubation, the reactions were quenched by the addition of 50 μl 30% acetic acid to each well.

The plates were then shaken for 1 hour at room temperature.

The plates were transferred to a TopCount NXT (TRADE MARK) (Packard, UK) for scintillation counting. Values recorded are counts per minute (cpm) following a 30 second counting of each well.

The % enzyme activity for each compound is then calculated using the following equation:

$$\%\ \text{Inhibition} = 100 - \left(100 \times \frac{(\text{cpm of unknowns} - \text{mean negative cpm})}{(\text{mean positive cpm} - \text{mean neagative cpm})}\right)$$

$IC_{50}$ values (the concentration at which 50% of the enzyme activity is inhibited) were calculated, which are determined over a range of different concentrations, normally from 10 μM down to 0.001 μM. Such $IC_{50}$ values are used as comparative values to identify increased compound potencies.

All compounds tested had a $IC_{50}$ of less than 0.1 μM.

The following compounds have an $IC_{50}$ of less than 0.01μM: 2, 3, 4, 5 9, 10, 13, 14, 15, 16, 17, 18, 19, 20, 21, 25, 27, 28, 29, 31, 33, 34, 35, 37, 40, 41, 44, 46, 47, 48, 49, 51, 53, 73, 74, 75, 76, 77, 78, 79, 80, 83, 84, 85, 97, 98, 99, 101, 103, 105, 106, 107, 111, 112, 113, 114, 117, 118, 119, 120, 121, 122, 130, 131, 132, 133, 138, 139, 140, 143, 149, 153, 155, 156, 157, 159, 160, 161, 175, 176, 177, 178, 179, 181, 182, 186, 188, 190, 191, 195, 197, 198, 200, 201, 202, 203, 205, 206, 207, 208, 209, 210, 211, 212, 213, 214, 216, 217, 218, 219, 221, 222, 223, 225, 226, 227, 228, 229, 232, 237, 240, 243, 246, 256.

The following compounds, as well as those above, have an $IC_{50}$ of less than 0.02μM: 1, 6, 7, 8, 11, 12, 22, 23, 24, 26, 32, 36, 38, 39, 42, 43, 45, 50, 54, 55, 56, 58, 59, 81, 82, 86, 87, 88, 89, 90, 91, 92, 93, 94, 95, 96, 100, 104, 108, 109, 110, 115, 116, 123, 126, 127, 128, 134, 135, 137, 141, 145, 146, 147, 150, 151, 152, 154, 158, 162, 163, 174, 180, 185, 187, 189, 192, 193, 194, 196, 199, 204, 215, 220, 224, 230, 233, 234, 235, 239, 241, 242, 244, 250, 251, 258, 259 and 261.

The Potentiation Factor ($PF_{50}$) for compounds is calculated as a ratio of the $IC_{50}$ of control cell growth divided by the $IC_{50}$ of cell growth + PARP inhibitor. Growth inhibition curves for both control and compound treated cells are in the presence of the alkylating agent methyl methanesulfonate (MMS). The test compounds were used at a fixed concentration of 0.2 micromolar. The concentrations of MMS were over a range from 0 to 10 μg/ml.

Cell growth was assessed using the sulforhodamine B (SRB) assay (Skehan, P., et al., (1990) New colorimetric cytotoxicity assay for anticancer-drug screening. J. Natl. Cancer Inst. 82, 1107-1112.). 2,000 HeLa cells were seeded into each well of a flat-bottomed 96-well microtiter plate in a volume of 100 μl and incubated for 6 hours at 37°C. Cells were either replaced with media alone or with media containing PARP inhibitor at a final concentration of 0.5, 1 or 5 μM. Cells were allowed to grow for a further 1 hour before the addition of MMS at a range of concentrations (typically 0, 1, 2, 3, 5, 7 and 10 μg/ml) to either untreated cells or PARP inhibitor treated cells. Cells treated with PARP inhibitor alone were used to assess the growth inhibition by the PARP inhibitor.

Cells were left for a further 16 hours before replacing the media and allowing the cells to grow for a further 72 hours at 37°C. The media was then removed and the cells fixed with 100μl of ice cold 10% (w/v) trichloroacetic acid. The plates were incubated at 4°C for 20 minutes and then washed four times with water. Each well of cells was then stained with 100μl of 0.4% (w/v) SRB in 1% acetic acid for 20 minutes before washing four times with 1% acetic acid. Plates were then dried for 2 hours at room temperature. The dye from the stained cells was solubilized by the addition of 100μl of 10mM Tris Base into each well. Plates were gently shaken and left at room temperature for 30 minutes before measuring the optical density at 564nM on a Microquant microtiter plate reader.

All the compounds tested had a $PF_{50}$ at 200nM of at least 2.0.

**Example 21**

Materials and Methods

*Small molecule inhibitors of PARP:*

Compound (**4**) was dissolved in DMSO at 10mM and stored at -20°C in the dark.

*Cell Lines*

VC8 cells and the mouse Brca2 BAC complemented derivatives were as described in M. Kraakman-van der Zwet, et al., Mol Cell Biol 22, 669-79 (2002)). ES cells defective in Brca2 function have been described previously (Tutt,

et al., EMBO Rep 3, 255-60 (2002)). The construction of ES cells defective in Brcal will be described elsewhere but have previously been validated (Foray, et al., Embo J, 22, 2860-71 (2003)).

*Clonogenic Assays*

For measurement of cellular sensitivity to a PARP inhibitor (compound 4), cell cultures in exponential growth were trypsinised and seeded at various densities in 6-well plates onto Mitomycin C inactivated mouse embryonic fibroblasts and where appropriate treated with the test compound after 18 hours. For continuous exposure, cells were re-fed every 4 days with fresh medium and inhibitor. After 10-14 days, cells were washed with PBS, fixed in methanol and stained with crystal violet. Colonies containing greater than approximately 50 cells were counted. Experiments were performed at least three times in triplicate.

Results

*Reduction in the viability of BRCA1 and BRCA2 deficient cells*

Compound **4** was used to probe the sensitivity of cells deficient in Brca1 or Brca2 to the inhibition of PARP activity. Clonogenic assays showed that both Brca1 and Brca2 deficient cell lines were extremely sensitive to compound **4** compared to otherwise isogenic cells (Fig. 1A, 1B). The $SF_{50}$ (dosage at which 50% of cells survived) for Compound **4** was 1.5 x $10^{-8}$M for cells deficient in Brca1, whilst the $SF_{50}$ for matched wild type cells was 7 x $10^{-6}$M (Figure 1A). This represents a factor of 467 fold enhanced sensitivity of Brca1 mutant cells compared to wild type cells.

The $SF_{50}$ for Compound **4** was 1.2 x 10-8M for cells deficient in Brca2 whilst the $SF_{50}$ for matched wild type cells was 1.8 x $10^{-5}$M (Figure 1B). This represents a factor of 1,500 fold enhanced sensitivity of Brca2 mutant cells compared to wild type cells. Similar results were obtained with Chinese hamster ovary cells deficient in Brca2 (VC8) compared to a Brca2-complememted derivative (VC8-BAC)(Figure 2). The $SF_{50}$ for Compound **4** was 5 x $10^{-8}$M for the Brca2 deficient VC8 line whilst the $SF_{50}$ for matched control, VC8-BAC, was 3 x $10^{-5}$M (Figure 2). This represents a factor of 600 fold enhanced sensitivity of Brca2 mutant cells compared to wild type cells.

## Claims

**1.** A compound of the formula (I):

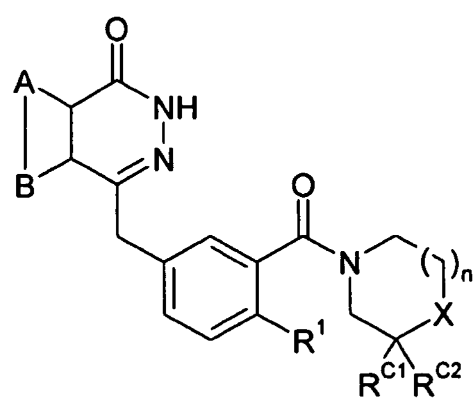

and isomers, salts and solvates thereof, wherein:

A and B together represent a fused benzene ring, optionally substituted by one or more groups selected from halo, nitro, hydroxy, ether, thiol, thioether, amino, $C_{1-7}$ alkyl, $C_{3-20}$ heterocyclyl and $C_{5-20}$ aryl;
X can be $NR^X$ or $CR^XR^Y$;
if X = $NR^X$ then n is 1 or 2 and if X = $CR^XR^Y$ then n is 1;
$R^X$ is selected from the group consisting of H, optionally substituted $C_{1-20}$ alkyl, $C_{5-20}$ aryl, $C_{3-20}$ heterocyclyl, amido, thioamido, ester, -C(=O)$R^Z$ , and sulfonyl groups, wherein the optional substituents are selected from $C_{1-20}$ alkyl, $C_{5-20}$ aryl, $C_{3-20}$ heterocyclyl, halo, hydroxy, ether, nitro, cyano, -C(=O)$R^Z$, carboxy, ester, amido, acylamido, ureido, acyloxy, thiol, thioether, sulfoxide, sulfonyl, thioamido and sulfonamino;

$R^Y$ is selected from H, hydroxy, amino;
or $R^X$ and $R^Y$ may together form a spiro-$C_{3-7}$ cycloalkyl or heterocyclyl group;
$R^{C1}$ and $R^{C2}$ are both hydrogen; and
$R^1$ is selected from H and halo,
wherein $R^Z$ is selected from H, $C_{1-7}$ alkyl, $C_{3-20}$ heterocyclyl or a $C_{5-20}$ aryl.

**2.** A compound according to claim 1, wherein $R^1$ is selected from H, Cl and F.

**3.** A compound according to either claim 1 or claim 2, wherein n is 2, X is $NR^X$, and $R^X$ is selected from the group consisting of: H; optionally substituted $C_{1-20}$ alkyl; optionally substituted $C_{5-20}$ aryl; optionally substituted ester groups; optionally substituted acyl groups; optionally substituted amido groups; optionally substituted thioamido groups; and optionally substituted sulfonyl groups.

**4.** A compound according to either claim 1 or claim 2, wherein n is 1, X is $NR^X$, and $R^X$ is selected from the group consisting of: H; optionally substituted $C_{1-20}$ alkyl; optionally substituted $C_{5-20}$ aryl; optionally substituted acyl; optionally substituted sulfonyl; optionally substituted amido; and optionally substituted thioamido groups.

**5.** A compound according to either claim 1 or claim 2, wherein n is 1, X is $CR^XR^Y$, $R^Y$ is H, and $R^X$ is selected from the group consisting of: H; optionally substituted $C_{1-20}$ alkyl; optionally substituted $C_{5-20}$ aryl; optionally substituted $C_{3-20}$ heterocyclyl; optionally substituted acyl; optionally substituted amido; and optionally substituted ester groups.

**6.** A compound according to claim 1, which is of formula (II):

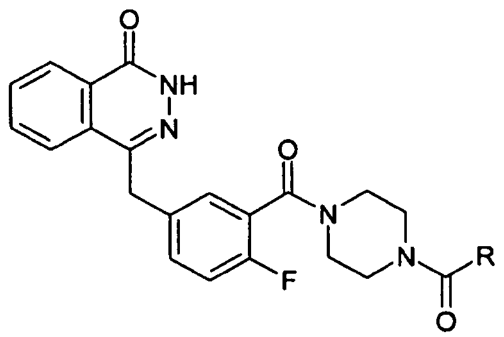

(II)

and isomers, salts and solvates thereof, where R is selected from:

a)

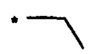

;

b)

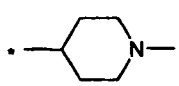

;

c)

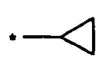

;

d)

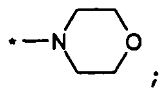

e)

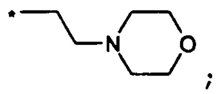

f)

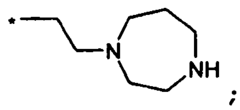

g)

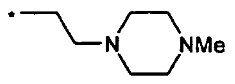

and
h)

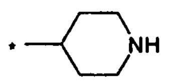

**7.** A compound according to claim 1, which is:

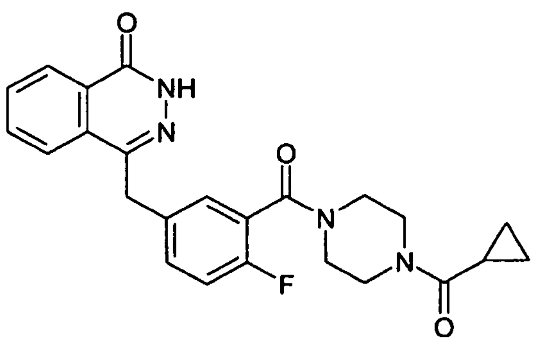

and isomers, salts and solvates thereof.

**8.** A compound:

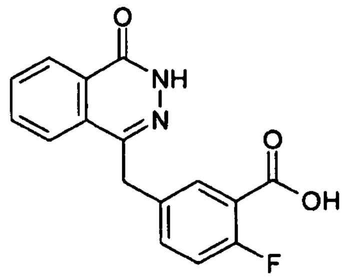

9. A pharmaceutical composition comprising a compound according to any one of claims 1 to 7 and a pharmaceutically acceptable carrier or diluent.

10. A compound according to any one of claims 1 to 7 for use in a method of treatment of the human or animal body.

11. The use of a compound according to any one of claims 1 to 7 in the preparation of a medicament for inhibiting the activity of PARP.

12. The use of a compound according to any one of claims 1 to 7 in the preparation of a medicament for the treatment of: vascular disease; septic shock; ischaemic injury; neurotoxicity; haemorraghic shock; viral infection; or diseases ameliorated by the inhibition of the activity of PARP.

13. The use of a compound according to any one of claims 1 to 7 in the preparation of a medicament for use as an adjunct in cancer therapy or for potentiating tumour cells for treatment with ionizing radiation or chemotherapeutic agents.

14. Use of a compound according to claims 1 to 7 in the manufacture of a medicament for use in the treatment of cancer in an individual, wherein said cancer is deficient in HR dependent DNA DSB repair pathway.

15. Use according to claim 14, wherein said cancer comprises one or more cancer cells having a reduced or abrogated ability to repair DNA DSB by HR relative to normal cells.

16. Use according to claim 15, wherein said cancer cells have a BRCA1 or BRCA2 deficient phenotype.

17. Use according to claim 16, wherein said cancer cells are deficient in BRCA1 or BRCA2.

18. Use according to any one of claims 14 to 17, wherein said individual is heterozygous for a mutation in a gene encoding a component of the HR dependent DNA DSB repair pathway.

19. Use according to claim 18, wherein said individual is heterozygous for a mutation in BRCA1 and/or BRCA2.

20. Use according to any one of the claims 14 to 19, wherein said cancer is breast, ovary, pancreas or prostate cancer.

21. Use according to any one of claims 14 to 20 wherein said treatment further comprises administration of ionising radiation or a chemotherapeutic agent.

## Patentansprüche

1. Verbindung der Formel (I):

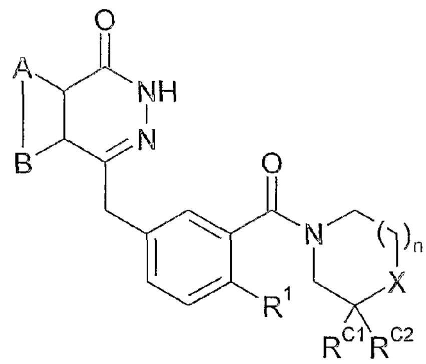

sowie Isomere, Salze und Solvate davon, worin:

A und B gemeinsam für einen kondensierten Benzolring stehen, der gegebenenfalls mit einer oder mehreren aus Halogen, Nitro, Hydroxy, Ether, Thiol, Thioether, Amino, $C_{1-7}$-Alkyl, $C_{3-20}$-Heterocyclyl und $C_{5-20}$-Aryl ausgewählten Gruppen substituiert ist;
X $NR^X$ oder $CR^XR^Y$ sein kann;
wenn X = $NR^X$ ist, gilt, dass n = 1 oder 2 ist, und wenn X = $CR^XR^Y$ ist, gilt, dass n = 1 ist;
$R^X$ aus der aus H, gegebenenfalls substituiertem $C_{1-20}$-Alkyl, $C_{5-20}$-Aryl, $C_{3-20}$-Heterocyclyl, Amido, Thioamido, Ester, -C(=O)$R^Z$ und Sulfonylgruppen bestehenden Gruppe ausgewählt ist, worin die optionalen Substituenten aus $C_{1-20}$-Alkyl, $C_{5-20}$-Aryl, $C_{3-20}$-Heterocyclyl, Halogen, Hydroxy, Ether, Nitro, Cyano, -C(=O)$R^Z$, Carboxy, Ester, Amido, Acylamido, Ureido, Acyloxy, Thiol, Thioether, Sulfoxid, Sulfonyl, Thioamido und Sulfonamido ausgewählt sind;
$R^Y$ aus H, Hydroxy und Amino ausgewählt ist;
oder $R^X$ und $R^Y$ gemeinsam gegebenenfalls eine Spiro-$C_{3-7}$-Cycloalkyl- oder -Heterocyclylgruppe bilden;
$R^{C1}$ und $R^{C2}$ beide Wasserstoff sind und
$R^1$ aus H und Halogen ausgewählt ist,
worin $R^Z$ aus H, $C_{1-7}$-Alkyl, $C_{3-20}$-Heterocyclyl und $C_{5-20}$-Aryl ausgewählt ist.

**2.** Verbindung nach Anspruch 1, worin $R^1$ aus H, Cl und F ausgewählt ist.

**3.** Verbindung nach Anspruch 1 oder 2, worin n = 2 ist, X = $NR^X$ ist und $R^X$ aus der aus H, gegebenenfalls substituiertem $C_{1-20}$-Alkyl, gegebenenfalls substituiertem $C_{5-20}$-Aryl, gegebenenfalls substituierten Estergruppen, gegebenenfalls substituierten Acylgruppen, gegebenenfalls substituierten Amidogruppen, gegebenenfalls substituierten Thioamidogruppen und gegebenenfalls substituierten Sulfonylgruppen bestehenden Gruppe ausgewählt ist.

**4.** Verbindung nach Anspruch 1 oder 2, worin n = 1 ist, X = $NR^X$ ist und $R^X$ aus der aus H, gegebenenfalls substituiertem $C_{1-20}$-Alkyl, gegebenenfalls substituiertem $C_{5-20}$-Aryl, gegebenenfalls substituierten Estergruppen, gegebenenfalls substituiertem Acyl, gegebenenfalls substituiertem Sulfonyl, gegebenenfalls substituiertem Amido und gegebenenfalls substituiertem Thioamido bestehenden Gruppe ausgewählt ist.

**5.** Verbindung nach Anspruch 1 oder 2, worin n = 1 ist, X = $CR^XR^Y$ ist, $R^Y$ = H ist und $R^X$ aus der aus H, gegebenenfalls substituiertem $C_{1-20}$-Alkyl, gegebenenfalls substituiertem $C_{5-20}$-Aryl, gegebenenfalls substituiertem $C_{3-20}$-Heterocyclyl, gegebenenfalls substituiertem Acyl, gegebenenfalls substituiertem Amido und gegebenenfalls substituierten Estergruppen bestehenden Gruppe ausgewählt ist.

**6.** Verbindung nach Anspruch 1, die der Formel (II) entspricht:

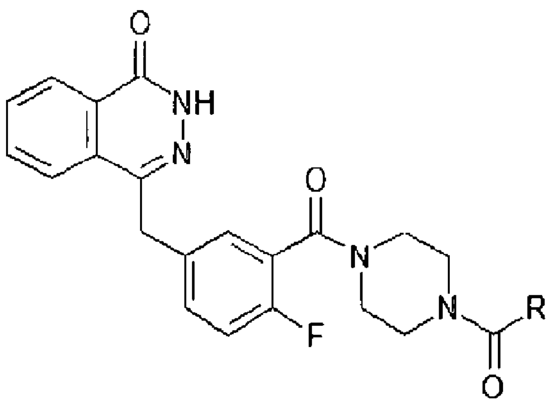

(II)

sowie Isomere, Salze und Solvate davon, worin R aus Folgendem ausgewählt ist:

a)

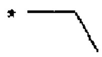

;

b)

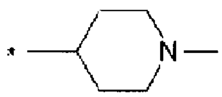

;

c)

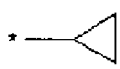

;

d)

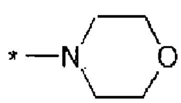

;

e)

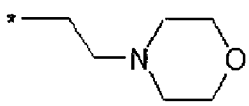

;

f)

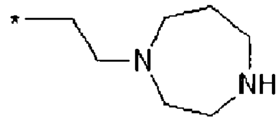

;

g)

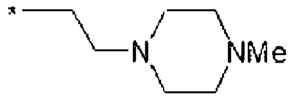

;

und

h)

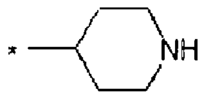

.

**7.** Verbindung nach Anspruch 1, nämlich

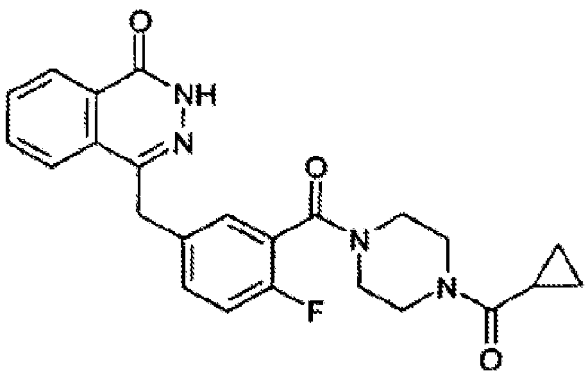

**8.** Die Verbindung:

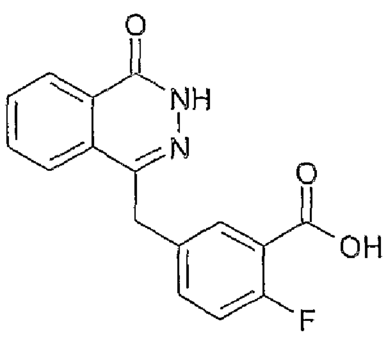

.

**9.** Pharmazeutische Zusammensetzung, die eine Verbindung nach einem der Ansprüche 1 bis 7 und einen pharmazeutisch annehmbaren Träger oder Verdünner umfasst.

**10.** Verbindung nach einem der Ansprüche 1 bis 7 zur Verwendung in einem Verfahren zur Behandlung des menschlichen oder tierischen Körpers.

**11.** Verwendung einer Verbindung nach einem der Ansprüche 1 bis 7 zur Herstellung eines Medikaments zur Hemmung der Aktivität von PARP.

**12.** Verwendung einer Verbindung nach einem der Ansprüche 1 bis 7 zur Herstellung eines Medikaments zur Behandlung einer Gefäßerkrankung, eines septischen Schocks, einer ischämischen Verletzung, von Neurotoxizität, eines hämorrhagischen Schocks, einer Virusinfektion oder von Erkrankungen, die durch die Hemmung der Aktivität von PARP gelindert werden.

**13.** Verwendung einer Verbindung nach einem der Ansprüche 1 bis 7 zur Herstellung eines Medikaments zur Verwendung als Hilfsstoff in der Krebstherapie oder zum Potenzieren von Tumorzellen zur Behandlung mit ionisierender Strahlung oder chemotherapeutischen Mitteln.

**14.** Verwendung einer Verbindung nach einem der Ansprüche 1 bis 7 zur Herstellung eines Medikaments zur Verwendung zur Behandlung von Krebs bei einem Individuum, wobei der Krebs defizient bezüglich des HR-abhängigen DNA-DSB-Reparaturwegs ist.

**15.** Verwendung nach Anspruch 14, worin der Krebs eine oder mehrere Krebszellen umfasst, die im Vergleich mit normalen Zellen eine verringerte oder aufgehobene Fähigkeit zur Reparatur von DNA-DSB mittels HR aufweisen.

**16.** Verwendung nach Anspruch 15, worin die Krebszellen einen BRCA1- oder BRCA2-defizienten Phänotyp aufweisen.

**17.** Verwendung nach Anspruch 16, worin die Krebszellen in Bezug auf BRCA1 oder BRCA2 defizient sind.

**18.** Verwendung nach einem der Ansprüche 14 bis 17, worin das Individuum heterozygot in Bezug auf eine Mutation in einem Gen ist, das für eine Komponente des HR-abhängigen DNA-DSB-Reparaturwegs kodiert.

**19.** Verwendung nach Anspruch 18, worin das Individuum heterozygot in Bezug auf eine Mutation in BRCA1 und/oder BRCA2 ist.

**20.** Verwendung nach einem der Ansprüche 14 bis 19, worin der Krebs Brust-, Eierstock-, Bauchspeicheldrüsen- oder Prostatakrebs ist.

**21.** Verwendung nach einem der Ansprüche 14 bis 20, worin die Behandlung weiters die Verabreichung ionisierender Strahlung oder eines chemotherapeutischen Mittels umfasst.

## Revendications

**1.** Composé de la formule (I) :

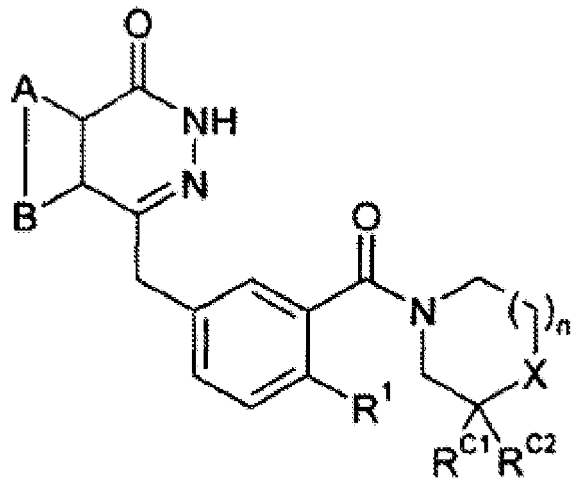

et des isomères, sels et solvates de celui-ci, où:

A et B ensemble représentent un cycle de benzène fusionné, optionnellement substitué par un ou plusieurs groupes sélectionnés parmi halo, nitro, hydroxy, éther, thiol, thioéther, amino, alkyle$C_{1-7}$, hétérocyclyle$C_{3-20}$ et aryle$C_{5-20}$;
X peut être $NR^x$ ou $CR^xR^y$;
si X = $NR^x$ alors n est 1 ou 2 et si X =$CR^xR^y$, alors n est 1;
$R^x$ est sélectionné dans le groupe consistant en H, optionnellement substitué par des groupes alkyle$C_{1-20}$, aryle$C_{5-20}$, hétérocyclyle$C_{3-20}$, amido, thioamido, ester, -C(-O)$R^z$ et sulfonyle, où les substituants optionnels sont sélectionnés parmi alkyle$C_{1-20}$, aryle $C_{5-20}$, hétérocyclyle $C_{3-20}$, halo, hydroxy, éther, nitro, cyano, -C(=O) $R^z$, carboxy, ester, amido, acylamido, uréido, acyloxy, thiol, thioéther, sulfoxyde, sulfonyle, thioamido et sulfonamino;
$R^y$ est sélectionné parmi H, hydroxy, amino;
ou $R^x$ et $R^y$ peuvent former ensemble un groupe cycloalkyle ou hétérocyclyle spiro-$C_{3-7}$;
$R^{C1}$ et $R^{C2}$ sont tous les deux hydrogène; et
$R^1$ est sélectionné parmi H et halo,
où $R^z$ est sélectionné parmi H, alkyle$C_{1-7}$, hétérocyclyle$C_{3-20}$ ou un aryle$C_{5-20}$ .

**2.** Composé selon la revendication 1, où $R^1$ est sélectionné parmi H, Cl et F.

**3.** Composé selon l'une quelconque des revendications 1 ou 2, où n est 2, X est $NR^x$, et $R^x$ est sélectionné dans le groupe consistant en: H; alkyle$C_{1-20}$ optionnellement substitué; aryle$C_{5-20}$ optionnellement substitué; des groupes ester optionnellement substitués; des groupes acyle optionnellement substitués; des groupes amido optionnellement substitués; des groupes thioamido optionnellement substitués; et des groupes sulfonyles optionnellement substitués.

**4.** Composé selon l'une quelconque des revendications 1 ou 2, où n est 1, X est $NR^x$ et $R^x$ est sélectionné dans le groupe consistant en: H; alkyle$C_{1-20}$ optionnellement substitué; aryle$C_{5-20}$ optionnellement substitué; acyle optionnellement substitué; sulfonyle optionnellement substitué; amido optionnellement substitué; et des groupes thioamido optionnellement substitués.

**5.** Composé selon l'une quelconque des revendications 1 ou 2, où n est 1, X est $CR^xR^y$, $R^y$ est H, et $R^x$ est sélectionné dans le groupe consistant en: H; alkyle$C_{1-20}$ optionnellement substitué; aryle$C_{5-20}$ optionnellement substitué; hétérocyclyle$C_{3-20}$ optionnellement substitué; acyle optionnellement substitué; amido optionnellement substitué; et des groupes ester optionnellement substitués.

**6.** Composé selon la revendication 1, qui est de la formule (II):

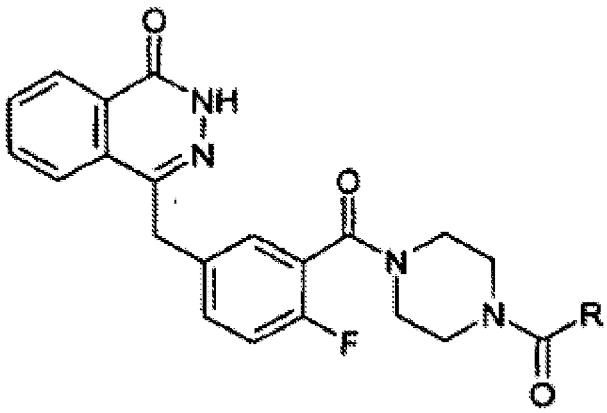

(II)

et des isomères, sels et solvates de celui-ci, où R est sélectionné parmi:

a)

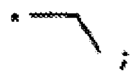

b)

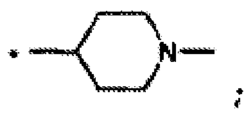

c)

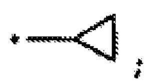

d)

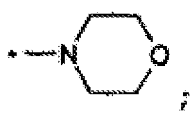

e)

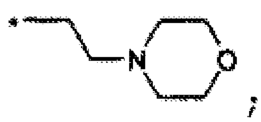

f)

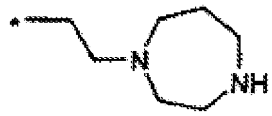
;

g)

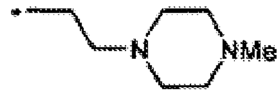
;

et
h)

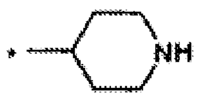
.

**7.** Composé selon la revendication 1, qui est:

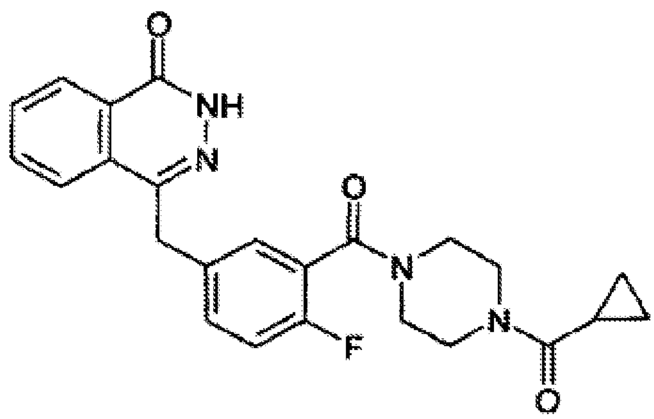

et des isomères, sels et solvates de celui-ci.

**8.** Composé:

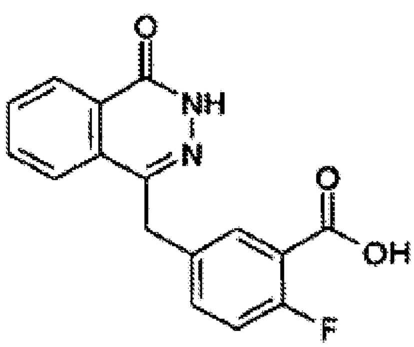

9. Composition pharmaceutique comprenant un composé selon l'une quelconque des revendications 1 à 7 et un support ou diluant pharmaceutiquement acceptable.

10. Composé selon l'une quelconque des revendications 1 à 7 pour utilisation dans une méthode de traitement du corps humain ou animal.

11. Utilisation d'un composé selon l'une quelconque des revendications 1 à 7 dans la préparation d'un médicament pour inhiber l'activité de PARP.

12. Utilisation d'un composé selon l'une quelconque des revendications 1 à 7 dans la préparation d'un médicament pour le traitement de: maladie vasculaire, choc septique; blessure ischémique; neurotoxicité; choc hémorragique; infection virale; ou maladies améliorées par l'inhibition de l'activité de PARP.

13. Utilisation d'un composé selon l'une quelconque des revendications 1 à 7 dans la préparation d'un médicament pour utilisation comme complément dans la thérapie du cancer ou pour la potentialisation des cellules tumorales pour le traitement avec un rayonnement ionisant ou des agents chimiothérapeutiques.

14. Utilisation d'un composé selon les revendications 1 à 7 dans la fabrication d'un médicament pour utilisation dans le traitement du cancer chez un individu, où ledit cancer est déficient dans la voie de réparation d'ADN DSB dépendante de la HR.

15. Utilisation selon la revendication 14, où ledit cancer comprend une ou plusieurs cellules cancéreuses ayant une aptitude réduite ou abrogée pour réparer ADN DSB par HR par rapport à des cellules normales.

16. Utilisation selon la revendication 15, dans laquelle lesdites cellules cancéreuses ont un phénotype de BRCA1 ou BRCA2 déficient.

17. Utilisation selon la revendication 16, dans laquelle lesdites cellules cancéreuses sont déficientes en BRCA1 ou BRCA2.

18. Utilisation selon l'une quelconque des revendications 14 à 17, dans laquelle ledit individu est hétérozygote pour une mutation dans un gène codant pour un composant de la voie de réparation d'ADN DSB dépendante de la HR.

19. Utilisation selon la revendication 18, où ledit individu est hétérozygote pour une mutation dans BRCA1 et/ou BRCA2.

20. Utilisation selon l'une quelconque des revendications 14 à 19, dans laquelle ledit cancer est le cancer du sein, des ovaires, du pancréas ou de la prostate.

21. Utilisation selon l'une quelconque des revendications 14 à 20, dans laquelle ledit traitement comprend en outre l'administration d'un rayonnement ionisant ou d'un agent chimiothérapeutique.

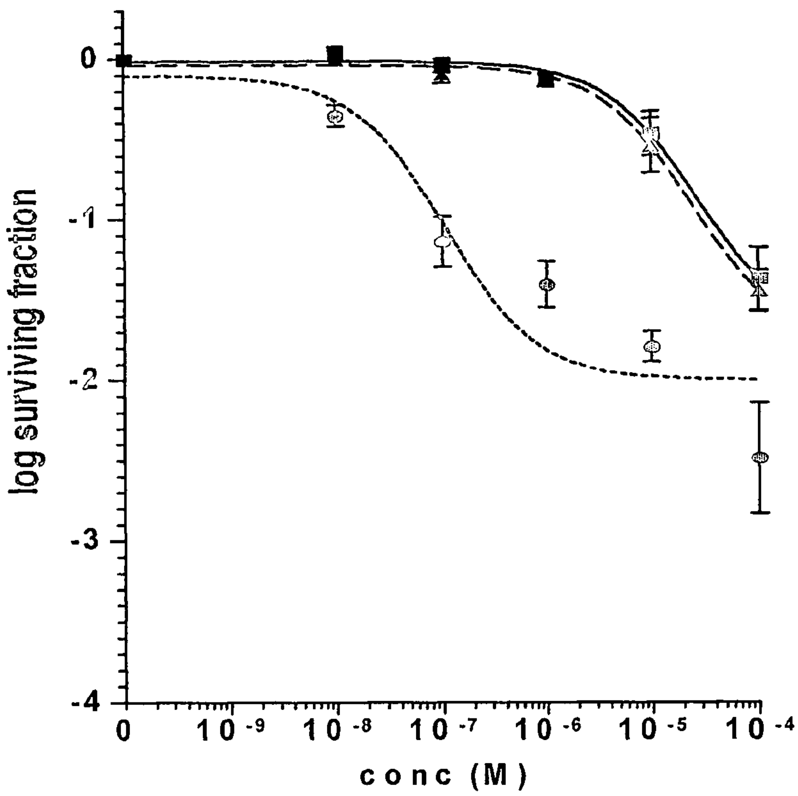
log surviving fraction
0
-1
-2
-3
-4
0
10 -9
10 -8
10 -7
10 -6
10 -5
10 -4
conc (M)
Fig. 1A

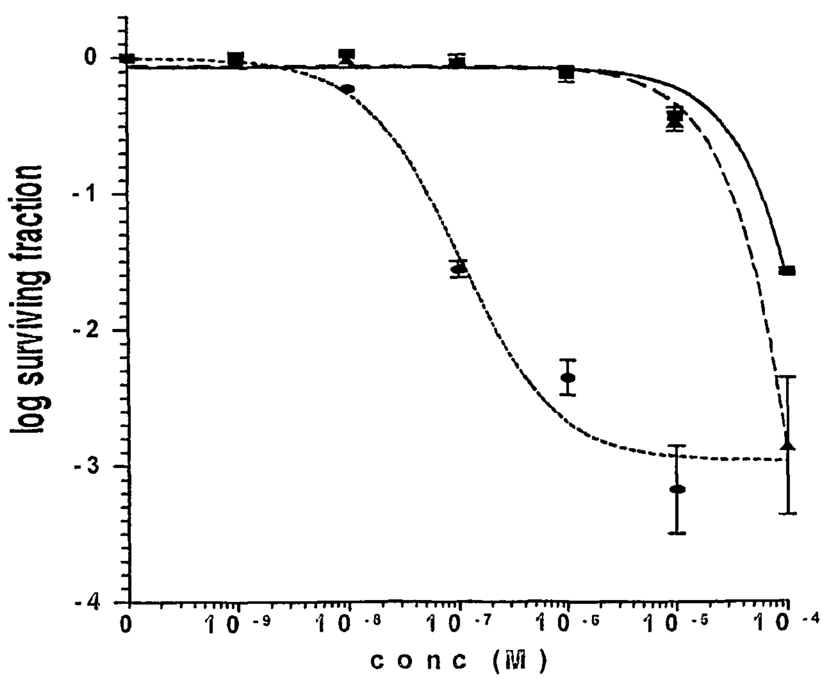
log surviving fraction
0
-1
-2
-3
-4
0
10 -9
10 -8
10 -7
10 -6
10 -5
10 -4
conc (M)

Fig. 1B

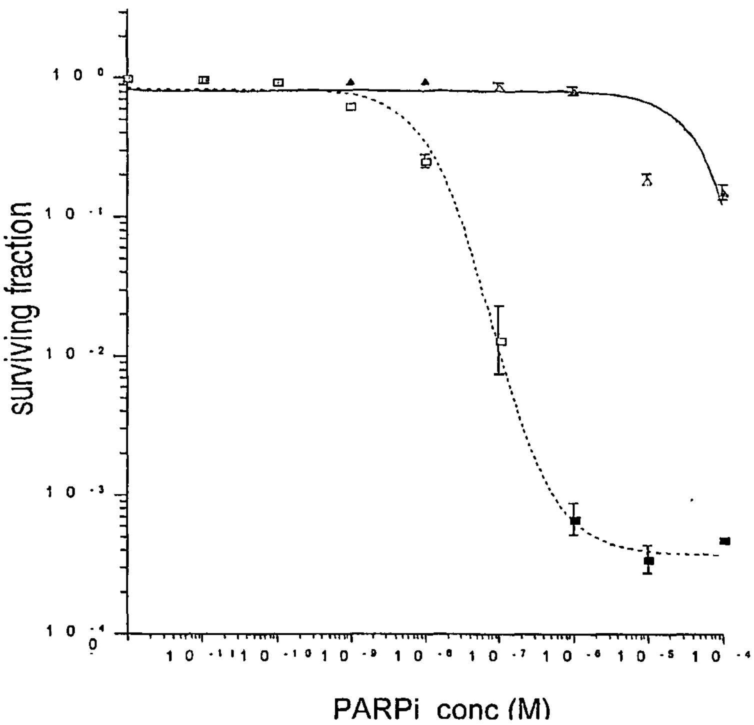
surviving fraction
1 0 ⁰
1 0 ⁻¹
1 0 ⁻²
1 0 ⁻³
1 0 ⁻⁴
0
1 0 ⁻¹¹ 1 0 ⁻¹⁰ 1 0 ⁻⁹ 1 0 ⁻⁸ 1 0 ⁻⁷ 1 0 ⁻⁶ 1 0 ⁻⁵ 1 0 ⁻⁴
PARPi conc (M)

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5032617 A
- US 5215738 A
- US 5091653 A
- WO 9118591 A
- WO 0236576 A
- EP 699754 A
- EP 705903 A
- WO 0226576 A


### Non-patent literature cited in the description


- **D'Amours et al.** *Biochem. J.,* 1999, vol. 342, 249-268
- **d'Adda di Fagagna et al.** *Nature Gen.,* 1999, vol. 23 (1), 76-80
- **Althaus, F.R. ; Richter, C.** ADP-Ribosylation of Proteins: Enzymology and Biological Significance. Springer-Verlag, 1987
- **Rhun et al.** *Biochem. Biophys. Res. Commun.,* 1998, vol. 245, 1-10
- **Miwa et al.** *Arch. Biochem. Biophys.,* 1977, vol. 181, 313-321
- **Burzio et al.** *Proc. Soc. Exp. Bioi. Med.,* 1975, vol. 149, 933-938
- **Hirai et al.** *Cancer Res.,* 1983, vol. 43, 3441-3446
- **Durkacz et al.** *Nature,* 1980, vol. 283, 593-596
- **Berger, N.A.** *Radiation Research,* 1985, vol. 101, 4-14
- **Ben-Hur et al.** *British Journal of Cancer,* 1984, vol. 49* (VI), 34-42
- **Schlicker et al.** *Int. J. Radiat. Bioi.,* 1999, vol. 75, 91-100
- **Wang et al.** *Genes Dev.,* 1995, vol. 9, 509-520
- **Menissier de Murcia et al.** *Proc. Natl. Acad. Sci. USA,* 1997, vol. 94, 7303-7307
- **Cantoni et al.** *Biochim. Biophys. Acta,* 1989, vol. 1014, 1-7
- **Szabo et al.** *J. Clin. Invest.,* 1997, vol. 100, 723-735
- **Cosi et al.** *J. Neurosci. Res.,* 1994, vol. 39, 38-46
- **Said et al.** *Proc. Natl. Acad. Sci. U.S.A.,* 1996, vol. 93, 4688-4692
- **Liaudet et al.** *Proc. Natl. Acad. Sci. U.S.A.,* 2000, vol. 97 (3), 10203-10208
- **Gaken et al.** *J. Virology,* 1996, vol. 70 (6), 3992-4000
- **Rattan ; Clark.** *Biochem. Biophys. Res. Comm.,* 1994, vol. 201 (2), 665-672
- **K.K. Khanna ; S.P. Jackson.** *Nat. Genet.,* 2001, vol. 27 (3), 247-254
- **Hughes-Davies et al.** *Cell,* vol. 115, 523-535
- **Wood et al.** *Science,* 2001, vol. 291, 1284-1289
- **Jasin M.** *Oncogene,* 2002, vol. 21 (58), 8981-93
- **Tutt et al.** *Trends Mol Med.,* 2002, vol. 8 (12), 571-6
- **Radice, P.J.** *Exp Clin Cancer Res,* 2002, vol. 21 (3), 9-12
- **Neuhausen, S.L. ; Ostrander, E.A.** *Genet. Test,* 1992, vol. 1, 75-83
- **Chappnis, P.O. ; Foulkes, W.D.** *Cancer Treat Res,* 2002, vol. 107, 29-59
- **Janatova M. et al.** *Neoplasma,* 2003, vol. 50 (4), 246-50
- **Jancarkova, N.** *Ceska Gynekol.,* 2003, vol. 68 (1), 11-6
- **Berge et al.** Pharmaceutically Acceptable Salts. *J. Pharm. Sci.,* 1977, vol. 66, 1-19
- Handbook of Pharmaceutical Additives. Synapse Information Resources, Inc, 2001
- Remington's Pharmaceutical Sciences. Lippincott, Williams & Wilkins, 2000
- Handbook of Pharmaceutical Excipients. 1994
- **Skehan, P. et al.** New colorimetric cytotoxicity assay for anticancer-drug screening. *J. Natl. Cancer Inst.,* 1990, vol. 82, 1107-1112
- **M. Kraakman-van der Zwet et al.** *Mol Cell Biol,* 2002, vol. 22, 669-79
- **Tutt et al.** *EMBO Rep,* 2002, vol. 3, 255-60
- **Foray et al.** *Embo J,* 2003, vol. 22, 2860-71